# EUROPEAN PATENT SPECIFICATION

(11) **EP 2 046 946 B1**
(45) Date of publication and mention of the grant of the patent: **26.10.2016**
(21) Application number: 07812340.3
(22) Date of filing: 26.06.2007
(51) Int. Cl.: C12N 5/00, C12N 5/073, C12N 5/0735

(54) **PLURIPOTENT STEM CELL CULTURE**
PLURIPOTENTE STAMMZELLENKULTUR
CULTURE DE CELLULES SOUCHES PLURIPOTENTES

(30) Priority: 26.06.2006 US 805770 P
(43) Date of publication of application: 15.04.2009
(73) Proprietor: Lifescan, Inc., Milpitas, CA 95035 (US)
(72) Inventor: PELLEGRINO-GENSEY, J. Lee, Ewing, New Jersey 08628 (US); FRYER, Benjamin, Philadelphia, Pennsylvania 19143 (US)
(74) Representative: Goodfellow, Hugh Robin
(86) International application number: PCT/US2007/072133
(87) International publication number: WO 2008/036447

(56) References cited:
- EP-A2- 2 302 036
- WO-A-01/51616
- WO-A-03/054169
- WO-A-2004/044158
- WO-A-2005/080551

## Description

### FIELD OF THE INVENTION

The present invention relates to the field of pluripotent stem cell culture media and to methods for culturing cells to produce such media. More particularly, the present invention provides methods and materials for propagating pluripotent stem cells in a substantially undifferentiated state, with and without a feeder layer.

### BACKGROUND

Pluripotent stem cells, such as, for example, embryonic stem cells have the ability to differentiate into all adult cell types. As such, embryonic stem cells may be a source of replacement cells and tissue for organs that have been damaged as a result of disease, infection, or congenital abnormalities. The potential for embryonic stem cells to be employed as a replacement cell source is hampered by the difficulty of propagating the cells *in vitro* while maintaining their pluripotency.

Current methods of culturing undifferentiated embryonic stem cells require complex culture conditions, such as, for example, culturing the embryonic stem cells in the presence of a feeder cell layer. Alternatively, media obtained by exposure to feeder cell cultures may be used to culture embryonic stem cells. Culture systems that employ these methods often use cells obtained from a different species than that of the stem cells being cultivated (xenogeneic cells). Additionally, these culture systems may be supplemented with animal serum.

For example, Reubinoff et al (Nature Biotechnology 18: 399 - 404 (2000)) and Thompson et al (Science 6 November 1998: Vol. 282. no. 5391, pp. 1145 - 1147) disclose the culture of embryonic stem cell lines from human blastocysts using a mouse embryonic fibroblast feeder cell layer.

In another example, WO2005014799 discloses conditioned medium for the maintenance, proliferation and differentiation of mammalian cells. WO2005014799 state: "The culture medium produced in accordance with the present invention is conditioned by the cell secretion activity of murine cells, in particular, those differentiated and immortalized transgenic hepatocytes, named MMH (Met Murine Hepatocyte)."

However, the use of xenogeneic cells, or xenogeneic cell products, increases the risk that the resulting embryonic stem cell populations produced by such methods may be contaminated.

Richards et al, (Stem Cells 21: 546-556, 2003) evaluated a panel of 11 different human adult, fetal and neonatal feeder cell layers for their ability to support human embryonic stem cell culture. Richards et al, states: "human embryonic stem cell lines cultured on adult skin fibroblast feeders retain human embryonic stem cell morphology and remain pluripotent".

US6642048 discloses media that support the growth of primate pluripotent stem (pPS) cells in feeder-free culture, and cell lines useful for production of such media. US6642048 states: "This invention includes mesenchymal and fibroblast-like cell lines obtained from embryonic tissue or differentiated from embryonic stem cells. Methods for deriving such cell lines, processing media, and growing stem cells using the conditioned media are described and illustrated in this disclosure."

US20020072117 discloses cell lines that produce media that support the growth of primate pluripotent stem cells in feeder-free culture. The cell lines employed are mesenchymal and fibroblast-like cell lines obtained from embryonic tissue or differentiated from embryonic stem cells. US20020072117 also discloses the use of the cell lines as a primary feeder cell layer.

In another example, Wang et al (Stem Cells 23: 1221-1227, 2005) discloses methods for the long-term growth of human embryonic stem cells on feeder cell layers derived from human embryonic stem cells.

In another example, Xu et al (Stem Cells 22: 972-980, 2004) discloses conditioned medium obtained from human embryonic stem cell derivatives that have been genetically modified to over express human telomerase reverse transcriptase.

In another example, Stojkovic et al (Stem Cells 2005 23: 306-314, 2005) disclose a feeder cell system derived from the spontaneous differentiation of human embryonic stem cells.

In a further example, Miyamoto et al (Stem Cells 22: 433-440,2004) disclose a source of feeder cells obtained from human placenta.

Amit et al (Biol. Reprod 68: 2150-2156, 2003) discloses a feeder cell layer derived from human foreskin.

In another example, Inzunza et al (Stem Cells 23: 544-549, 2005) disclose a feeder cell layer from human postnatal foreskin fibroblasts.

An alternative culture system employs serum-free medium supplemented with growth factors capable of promoting the proliferation of embryonic stem cells. For example, Cheon et al (BioReprod DOI:10.1095/biolreprod.105.046870, October 19, 2005) disclose a feeder-free, serum-free culture system in which embryonic stem cells are maintained in unconditioned serum replacement (SR) medium supplemented with different growth factors capable of triggering embryonic stem cell self-renewal.

In another example, Levenstein et al (Stem Cells 24: 568-574, 2006) disclose methods for the long-term culture of human embryonic stem cells in the absence of fibroblasts or conditioned medium, using media supplemented with bFGF.

In another example, US20050148070 discloses a method of culturing human embryonic stem cells in defined media without serum and without fibroblast feeder cells, the method comprising: culturing the stem cells in a culture medium containing albumin, amino acids, vitamins, minerals, at least one transferrin or transferrin substitute, at least one insulin or insulin substitute, the culture medium essentially free of mammalian fetal serum and containing at least about 100 ng/ml of a fibroblast growth factor capable of activating a fibroblast growth factor signaling receptor, wherein the growth factor is supplied from a source other than just a fibroblast feeder layer, the medium supported the proliferation of stem cells in an undifferentiated state without feeder cells or conditioned medium.

In another example, US20050233446 discloses a defined media useful in culturing stem cells, including undifferentiated primate primordial stem cells. In solution, the media is substantially isotonic as compared to the stem cells being cultured. In a given culture, the particular medium comprises a base medium and an amount of each of bFGF, insulin, and ascorbic acid necessary to support substantially undifferentiated growth of the primordial stem cells.

In another example, US6800480 states "In one embodiment, a cell culture medium for growing primate-derived primordial stem cells in a substantially undifferentiated state is provided which includes a low osmotic pressure, low endotoxin basic medium that is effective to support the growth of primate-derived primordial stem cells. The basic medium is combined with a nutrient serum effective to support the growth of primate-derived primordial stem cells and a substrate selected from the group consisting of feeder cells and an extracellular matrix component derived from feeder cells. The medium further includes non-essential amino acids, an anti-oxidant, and a first growth factor selected from the group consisting of nucleosides and a pyruvate salt."

In another example, US20050244962 states: "In one aspect the invention provides a method of culturing primate embryonic stem cells. One cultures the stem cells in a culture essentially free of mammalian fetal serum (preferably also essentially free of any animal serum) and in the presence of fibroblast growth factor that is supplied from a source other than just a fibroblast feeder layer. In a preferred form, the fibroblast feeder layer, previously required to sustain a stem cell culture, is rendered unnecessary by the addition of sufficient fibroblast growth factor."

In a further example, WO2005065354 discloses a defined, isotonic culture medium that is essentially feeder-free and serum-free, comprising: a. a basal medium; b. an amount of bFGF sufficient to support growth of substantially undifferentiated mammalian stem cells; c. an amount of insulin sufficient to support growth of substantially undifferentiated mammalian stem cells; and d. an amount of ascorbic acid sufficient to support growth of substantially undifferentiated mammalian stem cells.

In another example, WO2005086845 discloses a method for maintenance of an undifferentiated stem cell, said method comprising exposing a stem cell to a member of the transforming growth factor-beta (TGFβ) family of proteins, a member of the fibroblast growth factor (FGF) family of proteins, or nicotinamide (NIC) in an amount sufficient to maintain the cell in an undifferentiated state for a sufficient amount of time to achieve a desired result. WO 01/51616 discloses i.a. a method for culturing pluripotent stem cells using conditioned medium produced from a culture of feeder cells. The feeder cells are mouse embryonic feeder cells.

### SUMMARY

The present invention relates to the field of pluripotent stem cell culture and to methods for culturing cells to produce conditioned media and feeder cells.

The present invention provides a method to generate conditioned medium for the propagation of pluripotent stem cells, comprising the steps of:
a) culturing cells that supply the conditioning factors
b) adding a base medium to the cells that supply the conditioning factors,
c) exposing the base medium to the cells that supply the conditioning factors for a period of time sufficient to condition the medium, and
d) removing the conditioned medium;
wherein the cells that supply the conditioning factors are amniotic fluid-derived cells and express at least one of the following markers: HNF-1beta, HNF-3beta, SOX- 17, or GATA 6.

The present invention also provides methods and materials for propagating feeder cells for the propagation of pluripotent stem cells. The present invention also provides methods and materials for propagating pluripotent stem cells in a substantially undifferentiated state, with and without a feeder cell layer.

In one embodiment, the present invention provides methods to propagate human pluripotent stem cells without a feeder cell layer using a cell culture medium that has been conditioned by exposure to amniotic fluid-derived cells.

In an alternate embodiment, the present invention provides methods to propagate undifferentiated pluripotent stem cells without a feeder cell layer using a cell culture medium that has been conditioned by exposure to amniotic fluid-derived cells.

In one embodiment, the present invention provides methods to generate conditioned media for the propagation of human pluripotent stem cells without a feeder cell layer.

In an alternate embodiment, the present invention provides methods to generate conditioned media for the propagation of undifferentiated human pluripotent stem cells without a feeder cell layer.

In one embodiment, the present invention provides methods to propagate human pluripotent stem cells using a feeder cell layer comprising amniotic fluid-derived cells.

In an alternate embodiment, the present invention provides methods to propagate undifferentiated human pluripotent stem cells using a feeder cell layer comprising amniotic fluid-derived cells.

In one embodiment, the amniotic fluid-derived cells do not express at least one of the following markers: HNF-3 beta, or SOX-17.

In the invention, the amniotic fluid-derived cells express at least one of the following markers: HNF-1 beta, HNF-3 beta, SOX-17, or GATA 6. In an alternate embodiment, the amniotic fluid-derived cells which express at least one of the following markers: HNF-1 beta, HNF-3 beta, SOX-17, or GATA 6, may not express at least one of the following protein markers: CD117, Oct-4 or Tra2-54.

In one embodiment, the present disclosure provides methods to propagate human pluripotent stem cells without a feeder cell layer using a cell culture medium that has been conditioned by exposure to postpartum cells isolated from umbilical cord tissue.

In an alternate embodiment, the present disclosure provides methods to propagate undifferentiated pluripotent stem cells without a feeder cell layer using a cell culture medium that has been conditioned by exposure to postpartum cells isolated from umbilical cord tissue.

In one embodiment, the present invention provides methods to generate conditioned media for the propagation of human pluripotent stem cells without a feeder cell layer.

In an alternate embodiment, the present invention provides methods to generate conditioned media for the propagation of undifferentiated human pluripotent stem cells without a feeder cell layer.

In one embodiment, the present disclosure provides methods to propagate human pluripotent stem cells using a feeder cell layer comprising postpartum cells isolated from umbilical cord tissue.

In one embodiment, the present disclosure provides methods to propagate human pluripotent stem cells using postpartum cells isolated from umbilical cord tissue as a feeder cell layer.

In an alternate embodiment, the present disclosure provides methods to propagate undifferentiated pluripotent stem cells using postpartum cells isolated from umbilical cord tissue as a feeder cell layer.

In one embodiment, the present disclosure provides methods to generate a feeder cell layer using postpartum cells isolated from umbilical cord tissue.

In one embodiment of the present disclosure, the postpartum cells isolated from umbilical cord tissue are isolated from umbilical cord tissue substantially free of blood, the cells are capable of self-renewal and expansion in culture and having the potential to differentiate into cells of other phenotypes, wherein the cell does not produce CD45, CD117, or telomerase.

In the disclosure, the postpartum cells isolated from umbilical cord tissue produce one or more of CD10, CD13, CD44, CD73, CD90, PDGFr-alpha, or HLA-A, B, C.

In another embodiment of the present disclosure, the postpartum cells isolated from umbilical cord tissue produce each of CD10, CD13, CD44, CD73, CD90, PDGFr-alpha, and HLA-A, B, C.

### BRIEF DESCRIPTION OF THE DRAWINGS

**Figure 1** shows the morphology of the human embryonic stem cell line H9 at passage number 74, which was grown on MATRIGEL in MEF-CM for seven passages.
**Figure 2** shows the expression of pluripotency markers by immunocytochemistry of human embryonic stem cell line H9 at passage75 cultured in MEF-CM for eight passages.
**Figure 3** shows the expression of pluripotency markers by immunocytochemistry of human embryonic stem cell line H9 at passage75 cultured in AF1-CM for five passages.
**Figure 4** shows the expression of pluripotency markers by immunocytochemistry of human embryonic stem cell line H9 at passage75 cultured in AF2-CM for five passages.
**Figure 5** shows the morphology of the human embryonic stem cell line H9 at passage number 75, which was grown on MATRIGEL in AF1-CM for five passages.
**Figure 6** shows the morphology of the human embryonic stem cell line H9 at passage number 75, which was grown on MATRIGEL in AF2-CM for five passages.
**Figure 7** shows the expression of pluripotency markers by immunocytochemistry of human embryonic stem cell line H9 at passage75 cultured in AF3-CM for six passages.
**Figure 8** shows the expression of pluripotency markers by immunocytochemistry of human embryonic stem cell line H9 at passage75 cultured in AF4-CM for six passages.
**Figure 9** shows the morphology of the human embryonic stem cell line H9 at passage number 78, which was grown on MATRIGEL in AF3-CM for nine passages.
**Figure 10** shows the morphology of the human embryonic stem cell line H9 at passage number 77, which was grown on MATRIGEL in AF4-CM for eight passages.
**Figure 11** shows the morphology of the human embryonic stem cell line H9 at passage number 48, which was grown on MATRIGEL in UTC-CM for three passages.
**Figure 12** shows the typical morphology of a human embryonic stem cell colony grown on an AF feeder cell layer of AFCA007 cells for 7 passages. Staining for each of the pluripotency markers (E-cadherin, Oct4, SSEA4, and Sox2) is shown below a nuclear stained (DAPI) micrograph of the same field to demonstrate the total numbers of cells in the field. Colony appearance, cellular morphology and staining patterns indicate that pluripotency is maintained in human embryonic stem cells grown on AFCA007 cell feeder cell layers.
**Figure 13** shows the typical morphology of a human embryonic stem cell colony grown on an AF feeder cell layer of AFCA009 cells for 5 passages. Staining for each of the pluripotency markers (E-cadherin, Oct4, SSEA4, and Sox2) is shown below a nuclear stained (DAPI) micrograph of the same field to demonstrate the total numbers of cells in the field. Colony appearance, cellular morphology and staining patterns indicate that pluripotency is maintained in human embryonic stem cells grown on AFCA009 cell feeder cell layers.
**Figure 14** shows the typical morphology of a human embryonic stem cell colony grown on an AF feeder cell layer of AFDX022 cells for 11 passages. Staining for each of the pluripotency markers (E-cadherin, Oct4, SSEA4, and Sox2) is shown below a nuclear stained (DAPI) micrograph of the same field to demonstrate the total numbers of cells in the field. Colony appearance, cellular morphology and staining patterns indicate that pluripotency is maintained in human embryonic stem cells grown on AFDX022 cell feeder cell layers.
**Figure 15** shows the typical morphology of a human embryonic stem cell colony grown on an AF feeder cell layer of AFDX025 cells for 11 passages. Staining for each of the pluripotency markers (E-cadherin, Oct4, SSEA4, and Sox2) is shown below a nuclear stained (DAPI) micrograph of the same field to demonstrate the total numbers of cells in the field. Colony appearance, cellular morphology and staining patterns indicate that pluripotency is maintained in human embryonic stem cells grown on AFDX025 cell feeder cell layers.

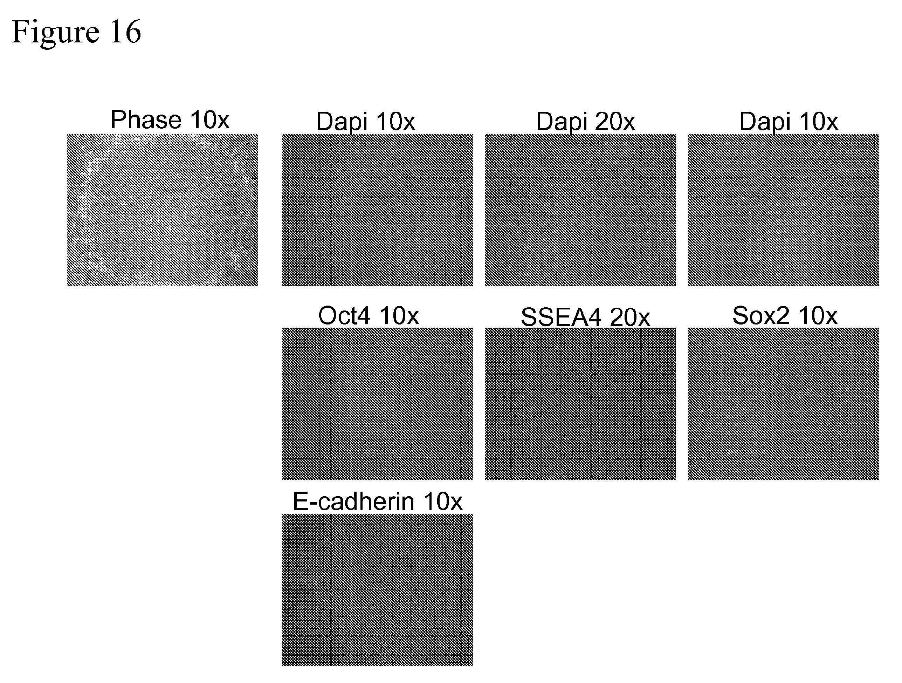

**Figure 16** shows the typical morphology of a human embryonic stem cell colony grown on a feeder cell layer of postpartum cells isolated from umbilical cord tissue for 12 passages. Staining for each of the pluripotency markers (E-cadherin, Oct4, SSEA4, and Sox2) is shown below a nuclear stained (DAPI) micrograph of the same field to demonstrate the total numbers of cells in the field. Colony appearance, cellular morphology and staining patterns indicate that pluripotency is maintained in human embryonic stem cells grown on a feeder cell layer of postpartum cells isolated from umbilical cord tissue.
**Figure 17** shows the formation of embryoid bodies formed from human embryonic stem cells that were cultured on an AF feeder cell layer of AFC3 [A], AFC4 [B], AF1 [C], AF2 [D], or UTC [E] feeders for 7-14 passages prior to embryoid body formation. Embryoid bodies were created by scraping or enzymatic detachment of embryonic stem cell clusters from the culture plate and seeded into low-attachment plastic dishes in DMEM-F12 supplemented with 20% FBS and cultured for 14 days, with media changes every 3 days.
**Figure 18** shows the morphology of BG01v cell line, cultured in MEF-CM at passage 32.
**Figure 19** shows the morphology of BG01v cell line, cultured in AF1-NF-CM for two passages.
**Figure 20** shows the morphology of BG01v cell line, cultured in AF2-NF-CM for two passages.
**Figure 21** shows the morphology of the BG01v cell line after nine passages in AF1-NF-CM.
**Figure 22** shows the morphology of the BG01v cell line after ten passages in AF2-NF-CM.
**Figure 23** shows the expression of pluripotency markers by immunocytochemistry of the BG01v cell line at passage35 cultured in MEF-CM.
Figure 24 shows the expression of pluripotency markers by immunocytochemistry of the BG01v cell line at passage35 cultured in AF2-NF-CM for five passages.

### DETAILED DESCRIPTION

For clarity of disclosure, and not by way of limitation, the detailed description of the invention is divided into the following subsections that describe or illustrate certain features, embodiments or applications of the present invention.

The present invention provides methods and materials for propagating pluripotent stem cells in a substantially undifferentiated state using conditioned media, wherein the media is conditioned using cells derived from amniotic fluid. Additionally, the present invention provides methods and materials for propagating pluripotent stem cells in a substantially undifferentiated state using a feeder cell layer comprising cells derived from amniotic fluid.

In another embodiment, the present disclosure provides methods and materials for propagating pluripotent stem cells in a substantially undifferentiated state using conditioned media, wherein the media is conditioned using cells derived from postpartum cells isolated from umbilical cord tissue. Additionally, the present disclosure provides methods and materials for propagating pluripotent stem cells in a substantially undifferentiated state using a feeder cell layer comprising postpartum cells isolated from umbilical cord tissue.

Stem cells are undifferentiated cells defined by their ability at the single cell level to both self-renew and differentiate to produce progeny cells, including self-renewing progenitors, non-renewing progenitors and terminally differentiated cells. Stem cells are also characterized by their ability to *differentiate in vitro* into functional cells of various cell lineages from multiple germ layers (endoderm, mesoderm and ectoderm), as well as to give rise to tissues of multiple germ layers following transplantation and to contribute substantially to most, if not all, tissues following injection into blastocysts.

Stem cells are classified by their developmental potential as: (1) totipotent, meaning able to give rise to all embryonic and extraembryonic cell types; (2) pluripotent, meaning able to give rise to all embryonic cell types; (3) multipotent, meaning able to give rise to a subset of cell lineages, but all within a particular tissue, organ, or physiological system (for example, hematopoietic stem cells (HSC) can produce progeny that include HSC (self- renewal), blood cell restricted oligopotent progenitors and all cell types and elements (e.g., platelets) that are normal components of the blood); (4) oligopotent, meaning able to give rise to a more restricted subset of cell lineages than multipotent stem cells; and (5) unipotent, meaning able to give rise to a single cell lineage (e.g. , spermatogenic stem cells).

Differentiation is the process by which an unspecialized ("uncommitted") or less specialized cell acquires the features of a specialized cell such as, for example, a nerve cell or a muscle cell. A differentiated or differentiation-induced cell is one that has taken on a more specialized ("committed") position within the lineage of a cell. The term committed, when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. De-differentiation refers to the process by which a cell reverts to a less specialized (or committed) position within the lineage of a cell. As used herein, the lineage of a cell defines the heredity of the cell, i.e., which cells it came from and what cells it can give rise to. The lineage of a cell places the cell within a hereditary scheme of development and differentiation. A lineage-specific marker refers to a characteristic specifically associated with the phenotype of cells of a lineage of interest and can be used to assess the differentiation of an uncommitted cell to the lineage of interest.

"Markers" as used herein, are nucleic acid or polypeptide molecules that are differentially expressed in a cell of interest. In this context, differential expression means an increased level for a positive marker and a decreased level for a negative marker. The detectable level of the marker nucleic acid or polypeptide is sufficiently higher or lower in the cells of interest compared to other cells, such that the cell of interest can be identified and distinguished from other cells using any of a variety of methods known in the art.

"Base medium" as used herein refers to a solution of salts and nutrients that is effective to support the growth of non-pluripotent cells in culture.

"Conditioned medium" as used herein refers to a base medium that is further supplemented with soluble factors derived from feeder cells.

"Feeder cells" as used herein refers to non-pluripotent stem cells on which pluripotent stem cells are plated. The non-pluripotent stem cells provide a milieu conducive to the growth of the plated pluripotent stem cells.

"Embryonic stem cells" or "ESCs" are cells obtained from morula or blastocyst stages of a pre-implantation stage embryo.

"Growth factor" as used for the purposes of describing the present invention refers to a substance that is effective to promote the growth of pluripotent stem cells that is not otherwise a component of the conditioned medium. Such substances include, but are not limited to, cytokines, chemokines, small molecules, neutralizing antibodies, and proteins.

### Amniotic Fluid-Derived Cells

Amniotic fluid derived cells have been routinely used for detecting chromosomal abnormality of the fetus. Amniotic fluid is typically sampled during the 2nd trimester (16 to 22 weeks of gestation). Previous art clearly demonstrates presence of three sub-population with distinct cell morphologies: "fibroblast cell-like" (F), "amniotic fluid cell-like" (AF) cells, and "epithelial cell-like" (E) cells. The F and AF cells rapidly expand whereas the E cells display a much slower growth curve and have poor clonal efficiency.

In one aspect of the present invention, amniotic fluid-derived cells are isolated by a multi-stage method, which essentially involves:
- Isolation of amniotic fluid,
- Centrifugation of the amniotic fluid, followed by removal of the supernatant,
- Resuspending the cell pellet in growth medium,
- Culturing the tissues and cells in a normoxic environment,
- Leaving the culture undisturbed for about 5 to 10 days without any media changes,
- Isolation of distinct colonies, and
- Culturing the isolated colonies in growth media.

In another aspect of the present invention, amniotic fluid-derived cells are isolated by a multi-stage method, which essentially involves:
- Isolation of amniotic fluid,
- Centrifugation of the amniotic fluid, followed by removal of the supernatant,
- Resuspending the cell pellet in growth medium,
- Culturing the tissues and cells in a normoxic environment,
- Leaving the culture undisturbed for about 5 to 10 days without any media changes,
- Isolation of distinct colonies using cloning rings,
- Culturing the isolated colonies in growth media
- Serial dilution cloning and identification of single cells that give rise to proliferating colonies, and
- Culturing the clones in growth media.

The cells may be isolated from the amniotic fluid of any animal. In one embodiment, the cells are isolated from a human amniotic fluid.

The culture plates may be pre-coated with agents such as, for example, fibronectin, vitronectin, laminin, collagen, gelatin, thrombospondin, placenta extracts, MATRIGEL™, tenascin, human serum, or combinations thereof.

If desirable, the amniotic fluid may be exposed, for example, to an agent (such as an antibody) that specifically recognizes a protein marker expressed by amniotic fluid cells, to identify and select amniotic fluid-derived cells, thereby obtaining a substantially pure population of amniotic fluid-derived cells.

Amniotic fluid-derived cells may be cultured in AMNIOMAX™ complete medium (Invitrogen). Alternatively, the cells may be cultured in Chang B/C medium (Irvine Scientific). Alternatively, the cells may be cultured in low glucose DMEM, supplemented with insulin-transferrin-selenium-X (ITS-X, Invitrogen, CA), 2% fetal bovine serum (FBS), 1% penicillin/streptomycin (P/S) + 25 ng/ml bFGF. Alternatively, the cells may be cultured in, DM-KNOCKOUT™ media (Invitrogen, CA), supplemented with 20% KNOCKOUT™ serum replacement (Invitrogen, CA), 10 ng/ml bFGF. Alternatively, the cells may be cultured in Williams' medium E supplemented with 2% defined FBS, 2mM L-glutamine, ITS, 55 µM 2-mercaptoethanol, 10ng/ml EGF, 4ng/ml bFGF, and 4ng/ml dexamethasone. Alternatively, the cells may be cultured in 1:1 DMEM-LG/MCDB 201, 2% FBS, ITS-X, β-mercaptoethanol 55 µM, 100 µM ascorbic acid-2-phosphate, 4ng/ml bFGF, 10ng/ml EGF, and 4ng/ml dexamethasone. Alternatively, the cells may be cultured in low glucose DMEM, supplemented with 20% FBS. Alternatively, the cell may be cultured in low glucose DMEM, supplemented with 5% FBS. The cells may also be cultured in low glucose DMEM/MCDB 201 medium (1:1), supplemented with 2% defined FBS, ITS-X, 1nM dexamethasone, 100 mM ascorbic acid 2-phosphate, 10ng/ml EGF, 10ng/ml PDGF-bb and 100 mM 2-mercaptoethanol. The media may be supplemented with bFGF, at concentrations from about 5 ng/ml to about 100 ng/ml. Alternatively, the cells may be cultured in 20% KNOCKOUT™ serum replacement + 80% KNOCKOUT™ DMEM, supplemented with 1 mM L-glutamine, 1% non-essential amino acids and 0.1 mM 2-mercaptoethanol. The medium may be conditioned overnight, on human or murine embryonic fibroblasts, human bone marrow derived stromal cells, or human placenta derived cells and supplemented with 4 ng/ml bFGF. Alternatively, the cells may be cultured in high glucose DMEM, supplemented with 20% defined FBS with 0.1 mM 2- mercaptoethanol.

In one embodiment, the amniotic fluid-derived cells have a fibroblast cell-like morphology and are isolated and cultured in DMEM-LG/MCDB201 50/50 + 2% FBS, ITS-X, β-mercaptoethanol, ascorbic acid-2-phosphate, 4ng/ml bFGF, 10ng/ml EGF, 4ng/ml dexamethasone on tissue culture plates treated with collagen type-I (Cell 1, Table I).

In an alternate embodiment, the amniotic fluid-derived cells have a fibroblast cell-like morphology and are isolated and cultured in Williams' E medium + 2% FBS, ITS, β-mercaptoethanol, 4ng/ml bFGF, 10ng/ml EGF, 4ng/ml dexamethasone (Cell 2, Table I).

In an alternate embodiment, the amniotic fluid-derived cells have an amniotic fluid cell-like morphology and are isolated and cultured according to the methods disclosed in US Patent Application 11/420895 (Cell 3 and Cell 4, Table I).

In one embodiment, the amniotic fluid-derived cells do not express at least one of the following markers: HNF-3 beta, or SOX-17.

In one embodiment, the amniotic fluid-derived cells express at least one of the following markers: HNF-1 beta, HNF-3 beta, SOX-17, or GATA 6. In an alternate embodiment, the amniotic fluid-derived cells which express at least one of the following markers: HNF-1 beta, HNF-3 beta, SOX-17, or GATA 6, may not express at least one of the following protein markers: CD 117, Oct-4 or Tra2-54.

Examples of other amniotic fluid-derived cell lines suitable for use in the present invention include, PCT application WO2003/042405 discloses isolation of c-Kit positive stem cells from chorionic villus, amniotic fluid and placenta (Cell 5, Table I).

In another example, U.S. Published Application 2005/0054093 discloses the isolation of stem cells from amniotic fluid. These cells express stage-specific embryonic antigen 3 (SSEA3), stage-specific embryonic antigen 4 (SSEA4), Tra1-60, Tra1-81, Tra2-54, Oct-4, HLA class I, CD13, CD44 CD49b and CD105 (Cell 6, Table I).

In another example, fetal cells have been isolated from amniotic fluid (in't Anker *et al*, *Blood* 102, 1548-1549,2003). The cells disclosed were positive for expression of the following markers: CD44, CD73, CD90, CD105, CD106, HLA-A, B, & C. The cells were negative for expression of the following markers: c-Kit (CD117), CD11, CD31, CD34, CD45 and HLA-D (Cell 7, Table I).

A population of mesenchymal stem cells isolated from amniotic fluid has also been reported in a publication to *Tsai et al (*Tsai et al, Human Reproduction 19, 1450-1456, 2004). The cells disclosed were positive for expression of the following markers: CD29, CD44, CD73, CD90, HLA-A, B, & C. The cells were also positive for the embryonic transcription factor Oct-4. The cells were negative for expression of the following markers: c-Kit (CD117), CD34 and HLA-D (Cell 8, Table I).

### Postpartum Cells Isolated from Umbilical Cord Tissue

Pluripotent cells may be isolated from umbilical cord tissue. Alternatively, cells may be isolated from Wharton's Jelly. Alternatively, cells may be isolated from umbilical vein or artery tissue. Alternatively, cells may be isolated from the umbilical matrix. Postpartum cells may be isolated from umbilical cord tissue by any suitable method in the art. The postpartum cells isolated from umbilical cord tissue of any animal. In one embodiment, the postpartum cells are isolated from a human umbilical cord.

In one embodiment of the present disclosure, the postpartum cells isolated from umbilical cord tissue are isolated according to the methods disclosed in U.S. Pat. No. 5,919,702.

In one embodiment of the present disclosure, the postpartum cells isolated from umbilical cord tissue are isolated according to the methods disclosed in U.S. Patent Application Publication US2003/0161818.

In one embodiment of the present disclosure, the postpartum cells isolated from umbilical cord tissue are isolated according to the methods disclosed in Mitchell et al. (Stem Cells 21:50-60, 2003).

In one embodiment of the present disclosure, the postpartum cells isolated from umbilical cord tissue are isolated according to the methods disclosed in Romanov et al. (Stem Cells 21(1):105-110, 2003).

In one embodiment of the present disclosure, the postpartum cells isolated from umbilical cord tissue are isolated according to methods disclosed in U.S. Patent Application No. 10/877,012. The postpartum cells isolated from umbilical cord tissue are isolated from umbilical cord tissue substantially free of blood, the cells are capable of self-renewal and expansion in culture and having the potential to differentiate into cells of other phenotypes, wherein the cell does not produce CD45, CD117, or telomerase. In one embodiment, the postpartum cells isolated from umbilical cord tissue produce one or more of CD10, CD 13, CD44, CD73, CD90, PDGFr-alpha, or HLA-A, B, C. In an alternate embodiment of the disclosure, the postpartum cells isolated from umbilical cord tissue produces each of CD10, CD13, CD44, CD73, CD90, PDGFr-alpha, and HLA-A, B, C.

### Generation of Conditioned Medium

Conditioned media described in this application are useful for propagating pluripotent stem cells in the presence or absence of feeder cells. However, the media (and the cells used to prepare media) have the characteristic of being able to support the growth of primate pluripotent stem cells in culture environments essentially free of feeder cells.

In one aspect of the present invention, conditioned medium is generated by a method which essentially involves:
- Culturing cells that supply conditioning factors,
- Adding a base medium to the cells,
- Exposing the base medium to the cells for a period of time sufficient to condition the medium, and
- Removing the conditioned medium.

In one aspect of the present invention, conditioned medium is generated by a method which essentially involves:
- Culturing cells that supply conditioning factors,
- Inactivating the cells,
- Replating the cells that supply conditioning factors,
- Adding a base medium to the cells,
- Exposing the base medium to the cells for a period of time sufficient to condition the medium,
- Removing the conditioned medium, and
- Purifying the conditioned medium.

The base medium used for conditioning can have any of several different formulae. The medium must be able to support the propagation of at least the cell line used for the conditioning of the medium. It is convenient that the medium also support the propagation of the pluripotent stem cells after conditioning. However, as an alternative, the medium can be supplemented with other factors or otherwise processed after conditioning to adapt it for propagating the pluripotent stem cells.

For propagating pluripotent stem cells in feeder-free culture, suitable base media can be made, from the following components, such as, for example, Dulbecco's modified Eagle's medium (DMEM), Gibco # 11965-092; Knockout Dulbecco's modified Eagle's medium (KO DMEM), Gibco # 10829-018; Ham's F12/50% DMEM basal medium; 200 mM L-glutamine, Gibco # 15039-027; non-essential amino acid solution, Gibco 11140-050; β- mercaptoethanol, Sigma # M7522; human recombinant basic fibroblast growth factor (bFGF), Gibco # 13256-029.

The base medium is combined with the cells used to condition the medium in an environment that allows the cells to release into the medium the components that propagate pluripotent stem cells. Optionally, the cells used to condition the medium can be inactivated (i.e., rendered incapable of substantial replication) by, for example, radiation, treatment with a chemical inactivator, such as, for example, mitomycin c, or by any other effective method. The inactivation of cells is not necessary in instances where the medium is separated from the conditioning cells before use in supporting pluripotent stem cell cultures.

The cells used to condition the medium are cultured in the medium for sufficient time to allow adequate concentration of released factors (or consumption of media components) to produce a medium that supports the propagation of pluripotent stem cells without differentiation. Typically, medium conditioned by culturing for 24 h at 37° C produces medium that supports pluripotent stem cell culture for 24 hours. However, the culturing period can be adjusted upwards or downwards, determining empirically (or by assaying for the concentration of essential factors) what constitutes an adequate period. After collecting a batch of conditioned medium, the cells can be used to condition a further batch of medium over a further culture period, for as many cycles as desired as long as the cells retain their ability to condition the medium in an adequate fashion.

The cells used to condition the medium are amniotic fluid-derived cells. Alternatively, in embodiments of the disclosure the cells used to condition the medium are postpartum cells isolated from umbilical tissue.

In one embodiment, the conditioned medium is supplemented with at least one additional factor. The additional factor may be a substance that the cell used to conditioned medium may have consumed in the conditioning process. Alternatively, the at least one factor may support the proliferation of the pluripotent stem cells. Alternatively, the at least one factor may promote, or inhibit the differentiation of the pluripotent stem cells. Examples of the at least one additional factor include, L-glutamine, bFGF, or LIF.

### Generation of a Feeder Cell Layer

Feeder cell layers described in this application are useful for propagating pluripotent stem cells.

In one aspect of the present invention, a feeder cell layer is generated by a method which essentially involves:
- Culturing cells that will form the feeder cell layer,
- Inactivating the cells,

The cells that will form the feeder cell layer may be cultured on a suitable culture substrate. In one embodiment, the suitable culture substrate is an extracellular matrix component, such as, for example, those derived from basement membrane or that may form part of adhesion molecule receptor-ligand couplings. In one embodiment, the suitable culture substrate is MATRIGEL® (Becton Dickenson). MATRIGEL® is a soluble preparation from Engelbreth-Holm-Swarm tumor cells that gels at room temperature to form a reconstituted basement membrane.

Other extracellular matrix components and component mixtures are suitable as an alternative. Depending on the cell type being proliferated, this may include laminin, gelatin, fibronectin, proteoglycan, entactin, heparan sulfate, and the like, alone or in various combinations.

The cells used to form the feeder cell layer may be inactivated (i.e., rendered incapable of substantial replication) by, for example, radiation, treatment with a chemical inactivator, such as, for example, mitomycin c, or by any other effective method.

The medium used for culturing the cells used to form the feeder cell layer can have any of several different formulae. The medium must be able to support the propagation of at least the cell line used to form the feeder cell layer. It is convenient that the medium also support the propagation of the pluripotent stem cells. However, as an alternative, the medium can be supplemented with other factors or otherwise processed to adapt it for propagating the pluripotent stem cells.

### Isolation, Expansion and Culture of Pluripotent Stem Cells

*Characterization of pluripotent stem cells:* Pluripotent stem cells may express one or more of the stage-specific embryonic antigens (SSEA) 3 and 4, and markers detectable using antibodies designated Tra-1-60 and Tra-1-81 (Thomson et al., Science 282:1145, 1998). Differentiation of pluripotent stem cells *in vitro* results in the loss of SSEA-4, Tra- 1-60, and Tra-1-81 expression (if present) and increased expression of SSEA-1. Undifferentiated pluripotent stem cells typically have alkaline phosphatase activity, which can be detected by fixing the cells with 4% paraformaldehyde, and then developing with Vector Red as a substrate, as described by the manufacturer (Vector Laboratories, Burlingame Calif.) Undifferentiated pluripotent stem cells also typically express Oct-4 and TERT, as detected by RT-PCR.

Another desirable phenotype of propagated pluripotent stem cells is a potential to differentiate into cells of all three germinal layers: endoderm, mesoderm, and ectoderm tissues. Pluripotency of pluripotent stem cells can be confirmed, for example, by injecting cells into severe combined immunodeficient (SCID) mice, fixing the teratomas that form using 4% paraformaldehyde, and then examining them histologically for evidence of cell types from the three germ layers. Alternatively, pluripotency may be determined by the creation of embryoid bodies and assessing the embryoid bodies for the presence of markers associated with the three germinal layers.

Propagated pluripotent stem cell lines may be karyotyped using a standard G-banding technique and compared to published karyotypes of the corresponding primate species. It is desirable to obtain cells that have a "norman karyotype," which means that the cells are euploid, wherein all human chromosomes are present and not noticeably altered.

*Sources ofpluripotent stem cells:* The types of pluripotent stem cells that may be used include established lines of pluripotent cells derived from tissue formed after gestation, including pre-embryonic tissue (such as, for example, a blastocyst), embryonic tissue, or fetal tissue taken any time during gestation, typically but not necessarily before approximately 10-12 weeks gestation. Non-limiting examples are established lines of human embryonic stem cells or human embryonic germ cells, such as, for example the human embryonic stem cell lines H1, H7, and H9 (WiCell). Also contemplated is use of the compositions of this disclosure during the initial establishment or stabilization of such cells, in which case the source cells would be primary pluripotent cells taken directly from the source tissues. Also suitable are cells taken from a pluripotent stem cell population already cultured in the absence of feeder cells. Also suitable are mutant human embryonic stem cell lines, such as, for example, BG01v (BresaGen, Athens, GA).

Pluripotent stem cells suitable for use in the present invention include, for example, the human embryonic stem cell line H9 (NIH code: WA09), the human embryonic stem cell line H1 (NIH code: WA01), the human embryonic stem cell line H7 (NIH code: WA07), and the human embryonic stem cell line SA002 (Cellartis, Sweden). Also suitable for use in the present invention are cells that express at least one of the following markers characteristic of pluripotent cells: ABCG2, cripto, CD9, FoxD3, Connexin43, Connexin45, Oct4, Sox2, Nanog, hTERT, UTF-1, ZFP42, SSEA-3, SSEA-4, Tra1-60, Tra1-81.

In one embodiment, human embryonic stem cells are prepared as described by Thomson et al. (U.S. Pat. No. 5,843,780; Science 282:1145, 1998; Curr. Top. Dev. Biol. 38:133 ff., 1998; Proc. Natl. Acad. Sci. U.S.A. 92:7844, 1995).

*Culture of pluripotent stem cells:* In one embodiment, pluripotent stem cells are typically cultured on a layer of feeder cells that support the pluripotent stem cells in various ways. Alternatively, pluripotent stem cells are cultured in a culture system that is essentially free of feeder cells, but nonetheless supports proliferation of pluripotent stem cells without undergoing substantial differentiation.

The pluripotent stem cells may be plated onto a suitable culture substrate. In one embodiment, the suitable culture substrate is an extracellular matrix component, such as, for example, those derived from basement membrane or that may form part of adhesion molecule receptor-ligand couplings. In one embodiment, the suitable culture substrate is MATRIGEL® (Becton Dickenson). MATRIGEL® is a soluble preparation from Engelbreth-Holm-Swarm tumor cells that gels at room temperature to form a reconstituted basement membrane.

Other extracellular matrix components and component mixtures are suitable as an alternative. Depending on the cell type being proliferated, this may include laminin, fibronectin, proteoglycan, entactin, heparan sulfate, and the like, alone or in various combinations.

The pluripotent stem cells may be plated onto the substrate in a suitable distribution and in the presence of a medium that promotes cell survival, propagation, and retention of the desirable characteristics. All these characteristics benefit from careful attention to the seeding distribution and can readily be determined by one of skill in the art.

Suitable culture media may be made from the following components, such as, for example, Dulbecco's modified Eagle's medium (DMEM), Gibco # 11965-092; Knockout Dulbecco's modified Eagle's medium (KO DMEM), Gibco # 10829-018; Ham's F12/50% DMEM basal medium; 200 mM L-glutamine, Gibco # 15039-027; non-essential amino acid solution, Gibco 11140-050; β- mercaptoethanol, Sigma # M7522; human recombinant basic fibroblast growth factor (bFGF), Gibco # 13256-029.

### Differentiation of Pluripotent Stem Cells

In one embodiment of the present invention, pluripotent stem cells are propagated in culture, while maintaining their pluripotency. Changes in pluripotency of the cells with time can be determined by detecting changes in the levels of expression of markers associated with pluripotency. Alternatively, changes in pluripotency can be monitored by detecting changes in the levels of expression of markers associated with differentiation, or markers associated with another cell type.

In an alternate embodiment, pluripotent stem cells are propagated in culture and then treated in a manner that promotes their differentiation into another cell type. The other cell type may be a cell expression markers characteristic of the definitive endoderm lineage. Alternatively, the cell type may be a cell expression markers characteristic of the pancreatic endoderm lineage. Alternatively, the cell type may be a cell expression markers characteristic of the pancreatic endocrine lineage. Alternatively, the cell type may be a cell expression markers characteristic of the β-cell lineage.

*Formation of Cells Expressing Markers Characteristic of the Definitive Endoderm Lineage:* Pluripotent stem cells may be differentiated into cells expressing markers characteristic of the definitive endoderm lineage by any method in the art.

For example, pluripotent stem cells may be differentiated into cells expressing markers characteristic of the definitive endoderm lineage according to the methods disclosed in D'Amour et al, Nature Biotechnology 23, 1534 - 1541 (2005).

For example, pluripotent stem cells may be differentiated into cells expressing markers characteristic of the definitive endoderm lineage according to the methods disclosed in Shinozaki et al, Development 131, 1651 - 1662 (2004).

For example, pluripotent stem cells may be differentiated into cells expressing markers characteristic of the definitive endoderm lineage according to the methods disclosed in McLean et al, Stem Cells 25, 29 - 38 (2007).

For example, pluripotent stem cells may be differentiated into cells expressing markers characteristic of the definitive endoderm lineage according to the methods disclosed in D'Amour et al, Nature Biotechnology 24, 1392 - 1401 (2006).

Markers characteristic of the definitive endoderm lineage are selected from the group consisting of SOX17, GATA4, Hnf-3beta, GSC, Cer1, Nodal, FGF8, Brachyury, Mix-like homeobox protein, FGF4 CD48, eomesodermin (EOMES), DKK4, FGF17, GATA6, CXCR4, C-Kit, CD99, and OTX2. Suitable for use in the present disclosure is a cell that expresses at least one of the markers characteristic of the definitive endoderm lineage. In one aspect of the present disclosure, a cell expressing markers characteristic of the definitive endoderm lineage is a primitive streak precursor cell. In an alternate aspect, a cell expressing markers characteristic of the definitive endoderm lineage is a mesendoderm cell. In an alternate aspect, a cell expressing markers characteristic of the definitive endoderm lineage is a definitive endoderm cell.

*Formation of Cells Expressing Markers Characteristic of the Pancreatic Endoderm Lineage:* Pluripotent stem cells may be differentiated into cells expressing markers characteristic of the pancreatic endoderm lineage by any method in the art.

For example, pluripotent stem cells may be differentiated into cells expressing markers characteristic of the pancreatic endoderm lineage according to the methods disclosed in D'Amour et al, Nature Biotechnology 24, 1392 - 1401 (2006).

Markers characteristic of the pancreatic endoderm lineage are selected from the group consisting of Pdxl, HNF-1beta, PTF1a, HNF-6, HB9 and PROX1. Suitable for use in the present disclosure is a cell that expresses at least one of the markers characteristic of the pancreatic endoderm lineage. In one aspect of the present disclosure, a cell expressing markers characteristic of the pancreatic endoderm lineage is a pancreatic endoderm cell.

*Formation of Cells Expressing Markers of the Pancreatic Endocrine Lineage:* Pluripotent stem cells may be differentiated into cells expressing markers characteristic of the pancreatic endocrine lineage by any method in the art.

For example, pluripotent stem cells may be differentiated into cells expressing markers characteristic of the pancreatic endocrine lineage according to the methods disclosed in D'Amour et al, Nature Biotechnology 24, 1392 - 1401 (2006).

For example, pluripotent stem cells may be differentiated into cells expressing markers characteristic of the pancreatic endocrine lineage, by the methods disclosed in Nature Biotechnology 24, 1392 - 1401 (2006).

For example, pluripotent stem cells may be differentiated into cells expressing markers characteristic of the pancreatic endocrine lineage, by the methods disclosed in D' Amour et al, Nature Biotechnology, 2006.

Markers characteristic of the pancreatic endocrine lineage are selected from the group consisting of NGN-3, NeuroD, Islet-1, Pdx-1, NKX6.1, Pax-4, Ngn-3, and PTF-1 alpha. In one embodiment, a pancreatic endocrine cell is capable of expressing at least one of the following hormones: insulin, glucagon, somatostatin, and pancreatic polypeptide. Suitable for use in the present disclosure is a cell that expresses at least one of the markers characteristic of the pancreatic endocrine lineage. In one aspect of the present disclosure, a cell expressing markers characteristic of the pancreatic endocrine lineage is a pancreatic endocrine cell. The pancreatic endocrine cell may be a pancreatic hormone expressing cell. Alternatively, the pancreatic endocrine cell may be a pancreatic hormone secreting cell.

In one aspect of the present disclosure, the pancreatic endocrine cell is a cell expressing markers characteristic of the β cell lineage. A cell expressing markers characteristic of the β cell lineage expresses Pdxl and at least one of the following transcription factors: NGN-3, Nkx2.2, Nkx6.1, NeuroD, Isl-1, HNF-3 beta, MAFA, Pax4, and Pax6. In one aspect of the present disclosure, a cell expressing markers characteristic of the β cell lineage is a β cell.

### Characterization of Cell Culture Media

In one embodiment of the present invention, condition medium is generated by exposing a base medium to amniotic fluid-derived cells. In an alternate embodiment of the disclosure, condition medium is generated by exposing a base medium to postpartum cells isolated from umbilical cord tissue. The conditioned medium may be purified from the cells and added to a pluripotent stem cell culture.

In one embodiment, the conditioned medium is analyzed and one, more than one, or all of its individual components characterized. Examples of methods suitable to characterize the components of cell culture medium are disclosed in Springer et al (Radiation Research: Vol. 164, No. 5, pp. 651-654, 2005).

In one embodiment, the concentrations of one, more than one, or all of the individual components are altered to optimize the conditioned medium. The optimization may be for the rate of proliferation of the pluripotent stem cells. Alternatively, the optimization may be for the maintenance of pluripotency. Alternatively, the optimization may be for the differentiation of the pluripotent stem cells into a specific cell type.

The present invention is further illustrated, but not limited by, the following examples.

### EXAMPLES

### Example 1

### The Establishment of Human Amniotic Fluid-Derived Cell Lines

Amniotic fluid used to isolate the cells of the present invention was taken from samples taken from routine amniocentesis performed at 16 to 22 weeks of gestation for fetal karyotyping. The amniotic fluid was centrifuged for 7 minutes at 400 *x* g and the supernatant removed. The resulting cell pellet was resuspended in growth media. The cells were cultured either on collagen type IV (1mg/100 mm plate), or on collagen type I (1 microgram/cm2), vitronectin (10 microgram/ml) or fibronectin (10 micrograms/ml) coated plates. The cell yield from amniotic fluid samples had a large variation (8000-300000 cell/sample) and some samples also contained a significant number of blood cell contamination. The cultures were left undisturbed for at least 5-10 days under hypoxic conditions (3% O₂). In parallel, cultures were established under similar conditions in normoxic conditions. Next, the cultures were fed with the same growth media and cultured until the cultures reached 70-80% confluency. Cells at this stage were referred to as "P0".

Cell lines 1 and 2 were isolated by mechanical dissociation of single colonies from the culture plates and were expanded by transferring the colonies into 24-well plates.

Cell line 1 was cultured in DMEM-LG/MCDB201 50/50 + 2% FBS, ITS-X, β-mercaptoethanol, ascorbic acid-2-phosphate, 4ng/ml bFGF, 10ng/ml EGF, 4ng/ml dexamethasone on tissue culture plates treated with collagen type-I and routinely passaged with TrypLE Express (Invitrogen). Cell line 2 was cultures in Williams' E medium + 2% FBS, ITS, β-mercaptoethanol, 4ng/ml bFGF, 10ng/ml EGF, 4ng/ml dexamethasone on fibronectin coated plates, and routinely passaged with TrypLE Express (Invitrogen).

In some cultures, colonies of cells were isolated by a cloning ring and sub cultured into a different culture plate. Cells were released from P0 culture by using TrypLE Express™ (Invitrogen) and seeded into fibronectin, vitronectin, or collagen type IV coated flaks/dishes/plates at various densities (50-10,000 cell/cm²). Some of the P0 cells were used for serial dilution cloning. The population doubling time of the fastest growing cells was approximately 24 hrs at early passages. The expanded cells were analyzed for markers by FACS **(Table I)** and PCR **(Table II).** Cells were typically split at -70% confluency and reseeded at 100-10000 cells/cm².

### Reference Example 2

### Culture of Human Embryonic Stem Cells in Media Conditioned with Mouse Embryonic Fibroblasts

Undifferentiated H9 human embryonic stem cells that had been maintained on inactivated primary mouse embryonic fibroblasts (MEF) were harvested and then maintained on MATRIGEL in conditioned medium from cultures of inactivated MEF.

*Preparation of conditioned medium (CM) from primary MEF:* Mouse embryonic fibroblasts maintained in DMEM-HG supplemented with 10% FBS and 1% non-essential amino acids were incubated for 2.5hr at 37°C in 0.1ml/cm² mitomycin C (0.01mg/ml in 80% DMEM-HG, 20% KSR, 2mM Glutamax, 1mM non-essential amino acids, 0.1mM mercaptoethanol, and 4ng/ml bFGF [human embryonic stem cell medium]), then washed five times with PBS before detaching with TrypLE Express. Pooled cells were washed four times in DMEM-HG supplemented with 10% FBS and 1% non-essential amino acids, then seeded on gelatin-coated flasks (0.1% gelatin in water for embryo transfer, 10ml/T75, one hour to overnight at 37°C) at 56,000/cm². After attaching overnight the medium was exchanged with human embryonic stem cell medium, using 0.5ml/cm². Every 24h the MEF-conditioned medium (MEF-CM) was harvested, filtered, and stored at -20°C. MEF-CM was harvested from inactivated cultures for 7 days. The MEF-CM was supplemented with fresh bFGF at 4ng/ml before use.

*MATRIGEL coating of tissue culture dishes:* Growth Factor Reduced MATRIGEL (Becton-Dickinson, Bedford Mass.) was thawed at 4°C, and then diluted 1:30 in cold DMEM/F12. Two mls of this solution was added to each 6cm dish, or 1ml to a well of a 6 well plate, and incubated 1hr at room temp. Plates were used within a few hours or stored at 4°C.

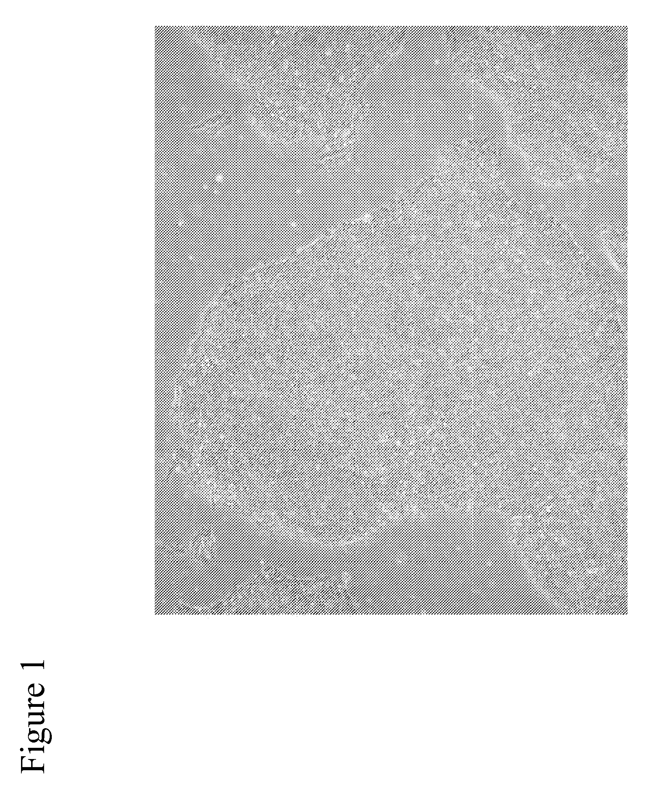

*Human embryonic stem cell culture:* Undifferentiated human embryonic stem cell colonies (H9, passage 67) were harvested from feeders by incubation in 1mg/ml collagenase IV in DMEM/F12 for 10 minutes, followed by scraping. Cell clumps were pelleted by centrifugation at 1000rpm for four minutes and the pellet dispersed gently with a 2ml pipet to break colonies into small clusters of cells. These cells were seeded into MATRIGEL-coated dishes in bFGF-supplemented MEF-CM, 50-150 colonies per 6cm dish in 5ml medium. Medium was changed daily. Colonies on MATRIGEL in MEF-CM became large and were passed when they occupied 70-80% of the surface area. The hES cells in the colonies had a high nucleus to cytoplasm ratio and had prominent nucleoli, similar to hES cells maintained on feeders (Figure **1**). Differentiated cells represented less than 10% of the cells in the culture.

For routine passage of cells in MEF-CM on MATRIGEL, cells were incubated in 1mg/ml collagenase IV in DMEM/F12 for up to 40 minutes and removed from the dishes by forceful streams of DMEM/F12 with minimal scraping. Cells were pelleted, dispersed, and seeded as above.

*Characterization of human Embryonic stem cells on MATRIGEL in MEF-CM:* H9 cells passaged onto MATRIGEL in MEF-CM at p68 were evaluated by FACS and immunostaining at p75 after repeated passage in conditioned medium. H9 cells were plated on MATRIGEL-coated glass and cultured in MEF-CM until 70% confluent. The cells were fixed in 4% paraformaldehyde and permeabilized in 0.5% Triton X-100 before incubation in 5% chicken serum to block non-specif interactions. Goat primary antibodies (goat anti-OCT4, R&D Systems AF1759; goat anti-nanog, R&D Systems AF1997; goat anti-Sox2, R&D Systems AF2018) were diluted 1:100 in 5% chicken serum and incubated one hour. After washing with PBS, Alexa-594-labeled chicken anti-goat IgG (Molecular Probes A21468) at 1:200 in 1% BSA in PBS was applied for 30 minutes followed by washes in PBS and 10 minute incubation in 1:10 diluted ProLong Gold DAPI/anti-fade reagent in PBS.

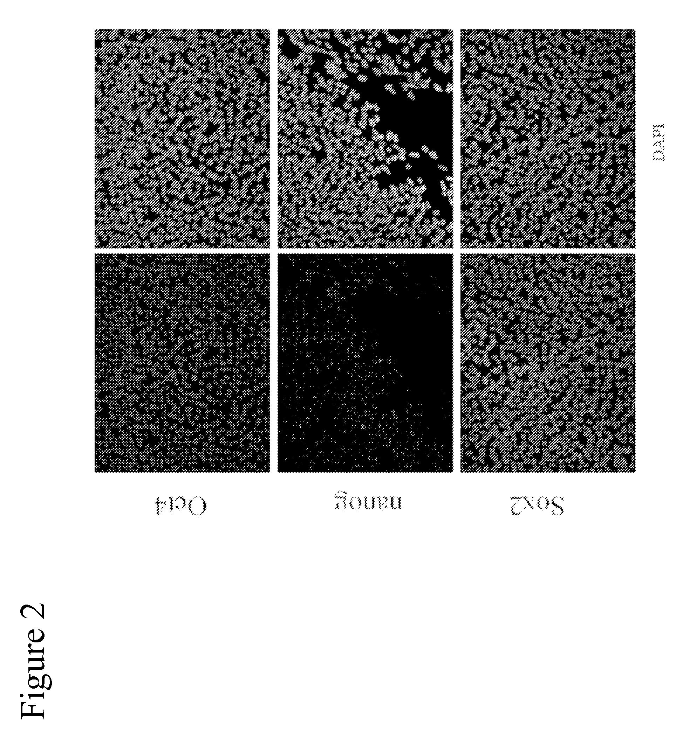

By FACS they were positive for SSEA-3, SSEA-4, TRA-1-60, and TRA-1-81, and negative for SSEA-1 **(Table III**). By immunostaining they were positive for nanog, Sox2, and Oct4 **(****Figure 2**).

H9 cells cultured in MEF-CM for 14 passages showed a normal karyotype (46, XX, **Table IV**).

### Reference Example 3

### Culture of Human Embryonic Stem Cells in Conditioned Medium from F-Cell-Like Morphology Amniotic Fluid-Derived Cells Maintains Markers of Human Embryonic Stem Cells

*Preparation of conditioned medium from Cell 1 (AF1-CM) and Cell 2 (AF2-CM):* Amniotic fluid-derived cells maintained in DMEM-HG supplemented with 10% FBS and 1% non-essential amino acids were incubated for 2.5hr at 37°C in 0.1ml/cm² mitomycin C (0.01mg/ml in DMEM-HG supplemented with 10% fetal bovine serum and 1% non-essential amino acids), then washed five times with PBS before detaching as single cells with TrypLE Express. Pooled cells were washed four times in DMEM-HG supplemented with 10% FBS and 1% non-essential amino acids, then seeded on gelatin-coated flasks (0.1% gelatin in water for embryo transfer, 10ml/T75, one hour to overnight at 37°C) at 56,000/cm². After attaching overnight the medium was exchanged with human embryonic stem cell medium, using 0.5ml/cm². Every 24h the amniotic fluid-derived cell-CM was harvested, supplemented with 2mM Glutamax, filtered, and stored at -20°C. Amniotic fluid-derived cell-CM was harvested from inactivated cultures for 7 days. The amniotic fluid-derived cell-CM was supplemented with fresh bFGF at 4ng/ml before use.

*Culture of human embryonic stem cells in amniotic fluid-derived cell-conditioned Medium:* On the day following passage of H9 cells in MEF-CM on MATRIGEL when colonies had attached and begun to proliferate, amniotic fluid-derived cell-CM was substituted for MEF-CM and changed daily. Cell passage was as in the previous example, with daily feedings of amniotic fluid-derived cell -CM instead of MEF-CM. The cells in the colonies had a high nucleus to cytoplasm ratio with some differentiated cells around the margins. Differentiated cells represented less than 10% of the cells in the culture. Cell proliferation paralleled that of cells in MEF-CM and cells in AF1-CM and AF2-CM were generally passed on the same day at the same split ratio as cells in MEF-CM.

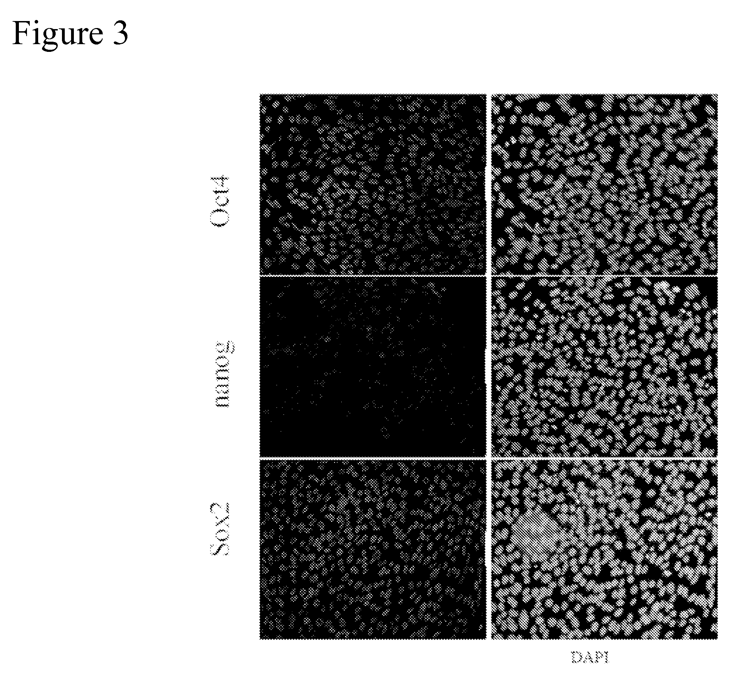

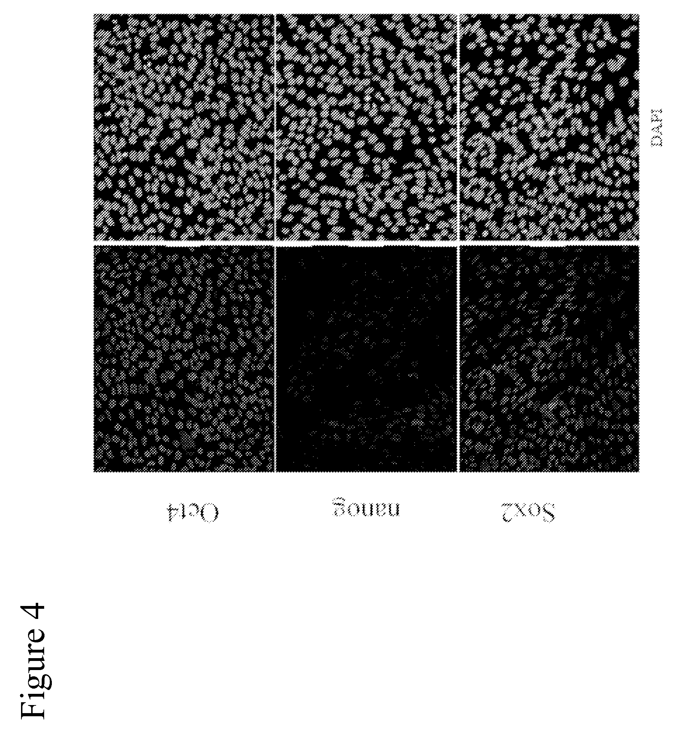

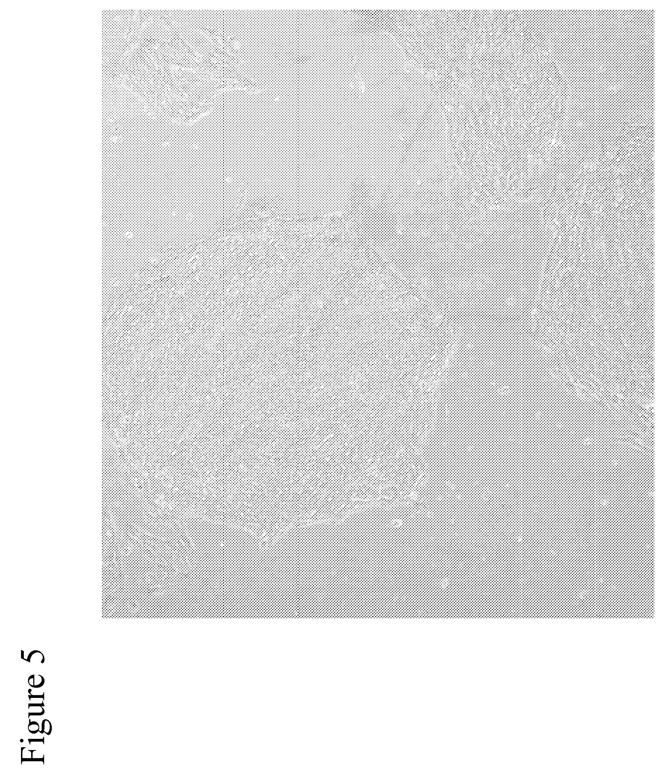

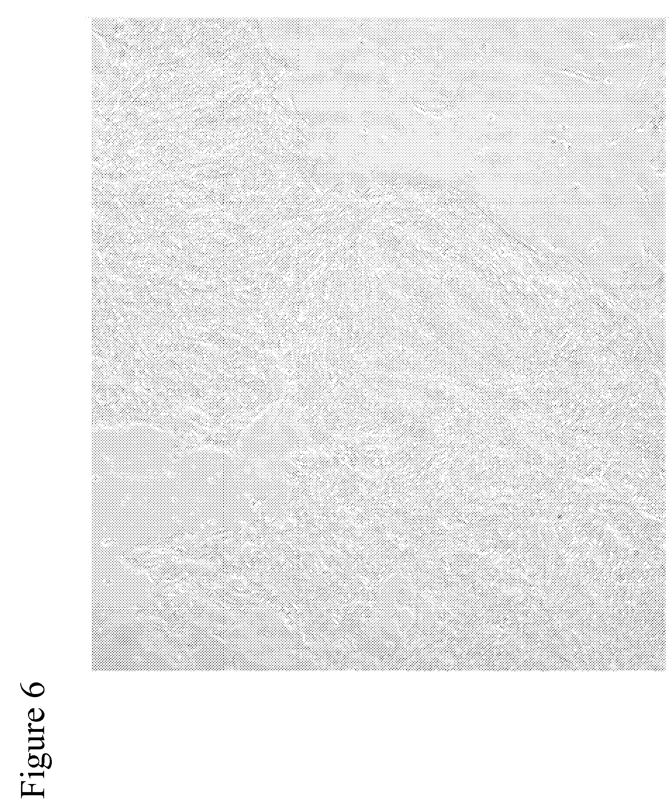

*Characterization of human embryonic stem cells on MATRIGEL in conditioned Medium obtained from Cell 1 and Cell 2:* Cells in their 5th passage in AF1-CM and AF2-CM and their 75th passage overall were evaluated by FACS and immunostaining. By FACS they were positive for SSEA-3, SSEA-4, TRA-1-60, and TRA-1-81, and negative for SSEA-1 **(Table III).** By immunostaining they were positive for nanog, Sox2, and Oct4 **(****Figures 3** **and** **4****).** Their morphology in each of the media is represented in **Figures 5** **and** **6****. Table V** shows expression of pluripotency markers in human embryonic stem cells cultured in AF1-CM and AF2-CM by real-time PCR. **Table VI** shows expression of differentiation markers in human embryonic stem cells cultured in AF1-CM and AF2-CM by real-time PCR.

### Reference Example 4

### Culture of Human Embryonic Stem Cells in Conditioned Medium from AF-Cell-Like Morphology Amniotic Fluid-Derived Cells Maintains Markers of Human Embryonic Stem Cells

*Preparation of conditioned medium from Cell 3 (AF3-CM) and Cell 4 (AF4-CM):* Amniotic fluid-derived cells maintained in DMEM-HG supplemented with 10% FBS and 1% non-essential amino acids were incubated for 2.5hr at 37°C in 0.1ml/cm² mitomycin C (0.01mg/ml in DMEM-HG supplemented with 10% FBS and 1% non-essential amino acids), then washed five times with PBS before detaching with TrypLE Express. Pooled cells were washed four times in DMEM-HG supplemented with 10% FBS and 1% non-essential amino acids, then seeded on gelatin-coated flasks (0.1% gelatin in water for embryo transfer, 10ml/T75, one hour to overnight at 37°C) at 56,000/cm². After attaching overnight the medium was exchanged with human embryonic stem cell medium, using 0.5ml/cm². Every 24h the AF-CM was harvested, supplemented with 2mM Glutamax, filtered, and stored at -20°C. AF-CM was harvested from inactivated cultures for 7 days. The AF-CM was supplemented with fresh bFGF at 4ng/ml before use.

*Culture of human embryonic stem cells in amniotic fluid-derived cell-conditioned Medium:* On the day following passage of H9 cells in MEF-CM on MATRIGEL when colonies had attached and begun to proliferate, AF3-CM or AF4-CM was substituted for MEF-CM and changed daily. Cell passage was as in the previous example, with daily feedings of AF-CM instead of MEF-CM. The cells in the colonies had a high nucleus to cytoplasm ratio with some differentiated cells around the margins. Differentiated cells represented less than 10% of the cells in the culture. Cell proliferation paralleled that of cells in MEF-CM and cells in AF3-CM and AF4-CM were generally passed on the same day at the same split ratio as cells in MEF-CM.

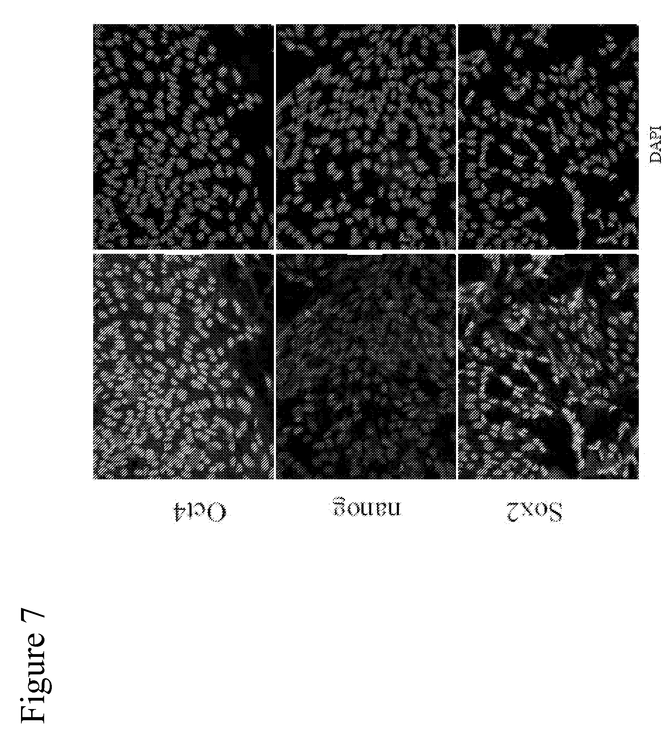

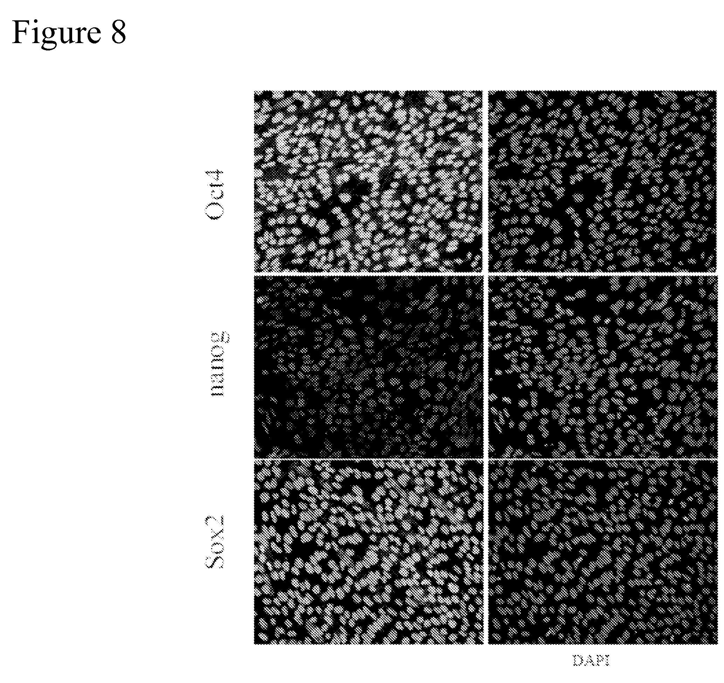

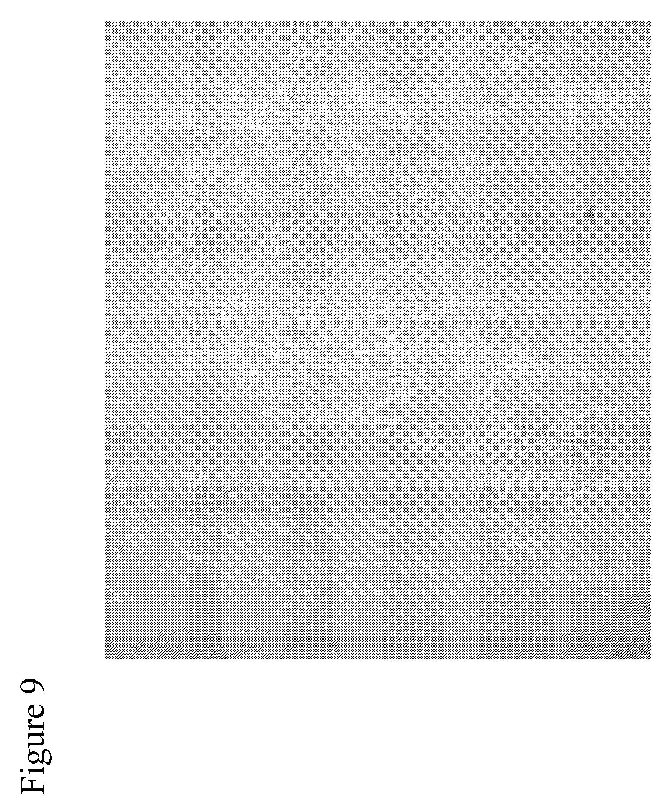

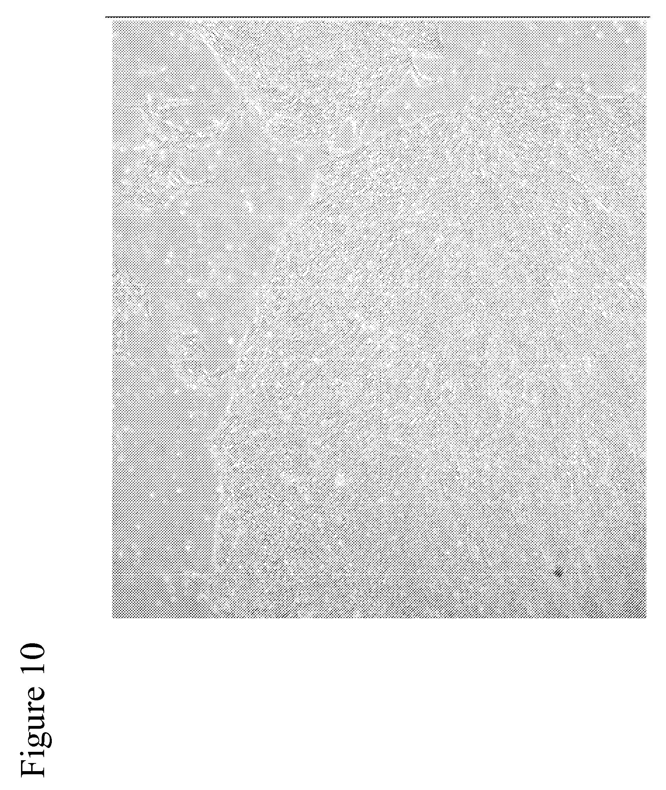

*Characterization of human embryonic stem cells on MATRIGEL in conditioned medium obtained from Cell 3 and Cell 4:* Cells in their 6th passage in AF3-CM and AF4-CM and their 78th passage overall were evaluated by FACS and immunostaining. By FACS they were positive for SSEA-3, SSEA-4, TRA-1-60, and TRA-1-81, and negative for SSEA-1 **(Table III). By** immunostaining they were positive for nanog, Sox2, and Oct4 **(****Figures 7** **and** **8****).** Their morphology in each of the media is represented in **Figures 9** **and** **10****.** H9 cells passed 9 times in AF3-CM or AF4-CM had normal autosomes and a modal chromosome number of 46 (46, XX, **Tables VII and VIII). Table IX** shows expression of pluripotency markers in human embryonic stem cells cultured in AF3-CM and AF4-CM by real-time PCR. **Table X** shows expression of differentiation markers in human embryonic stem cells cultured in AF3-CM and AF4-CM by real-time PCR.

### Reference Example 5

### Culture of Human Embryonic Stem Cells in Conditioned Medium from Postpartum Cells Isolated from Umbilical Cord Tissue Maintains Markers of Human Embryonic Stem Cells.

*Preparation of conditioned medium from Postpartum Cells Isolated from Umbilical Cord Tissue:* Postpartum cells isolated from umbilical cord tissue (UTCs) maintained in DMEM-F12 supplemented with 10% FBS were incubated for 2.5hr at 37°C in 0.1ml/cm² mitomycin C (0.01mg/ml in DMEM-F12 supplemented with 10% FBS), then washed five times with PBS before detaching with TrypLE Express. Pooled cells were washed four times in DMEM-F12 supplemented with 10% FBS, then seeded on gelatin-coated flasks (0.1% gelatin in water for embryo transfer, 10ml/T75, one hour to overnight at 37°C) at 56,000/cm². After attaching overnight the medium was exchanged with human embryonic stem cell medium, using 0.5ml/cm². Every 24h the UTC-CM was harvested, filtered, and stored at -20°C. UTC-CM was harvested from inactivated cultures for 7 days. The UTC-CM was supplemented with fresh bFGF at 20ng/ml before use.

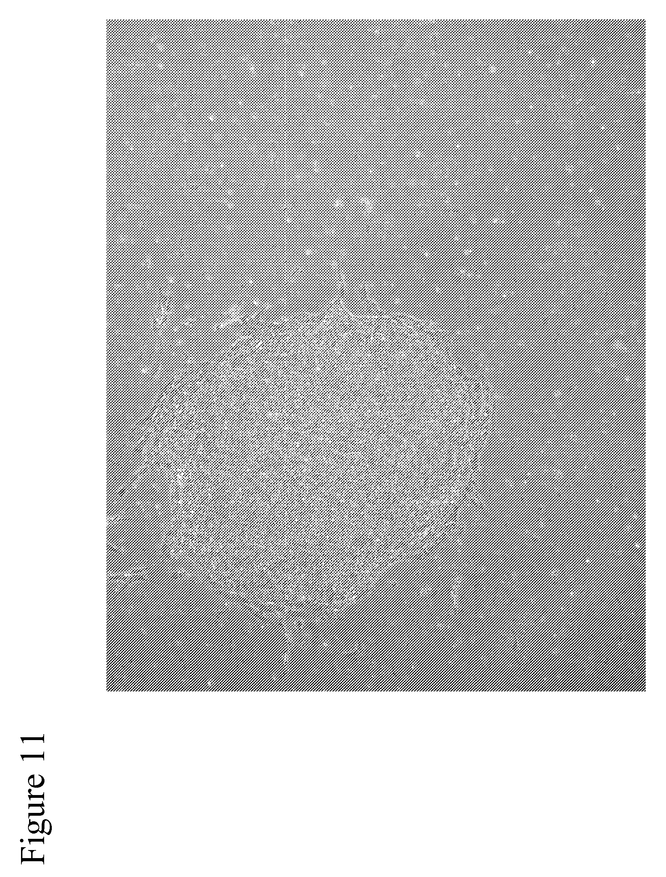

*Culture of human embryonic stem cells in conditioned medium from postpartum cells isolated from umbilical cord tissue:* On the day following passage of H9 cells in MEF-CM on MATRIGEL when colonies had attached and begun to proliferate, UTC-CM was substituted for MEF-CM and changed daily. Cell passage was as in the previous example, with daily feedings of UTC-CM instead of MEF-CM. The cells in the colonies had a high nucleus to cytoplasm ratio with some differentiated cells around the margins **(****Figure 11****).** Differentiated cells represented less than 10% of the cells in the culture. Cell proliferation paralleled that of cells in MEF-CM and cells in UTC-CM were generally passed on the same day at the same split ratio as cells in MEF-CM. time. Human embryonic stem cells cultured in UTC-CM exhibited the characteristic morphology of embryonic stem cells colonies and maintained expression of the pluripotency markers E-cadherin and Oct4 as measured by real time PCR while genes characteristic of differentiation such as Brachyury, N-Cadherin, CXCR4, Goosecoid, HNF-3b, Gata-4, and SOX-7 were not elevated **(Table XI).** These results indicate that human embryonic stem cells cultured in conditioned medium from postpartum cells isolated from umbilical cord tissue maintains markers of human embryonic stem cells.

### Reference Example 6

### Culture of Human Embryonic Stem Cells Using Amniotic Fluid-Derived Cells as a Feeder cell layer Maintains Markers of Human Embryonic Stem Cells.

Amniotic fluid cells of fibroblast or AF morphology were inactivated by mitomycin-C treatment as described above and seeded into gelatin-coated tissue culture plates at 10,000 - 50,000/cm² in DMEM-F12 or AMNIOMAX supplemented with 10% FBS and 1% non-essential amino acids and allowed to attach overnight or frozen in 10% DMSO/90% FBS and thawed at a later time. Plates of inactivated AF cells were washed with PBS and seeded with embryonic stem cells in hESC medium. The embryonic stem cell cultures were passaged onto freshly seeded inactivated AF cells in hESC medium for up to 17 passages. Human embryonic stem cells were examined for morphology, and expression of Sox2, Oct4, SSEA-1, SSEA-3, SSEA-4, TRA-1-60, and TRA-1-81. Karyotypic analysis was also performed.

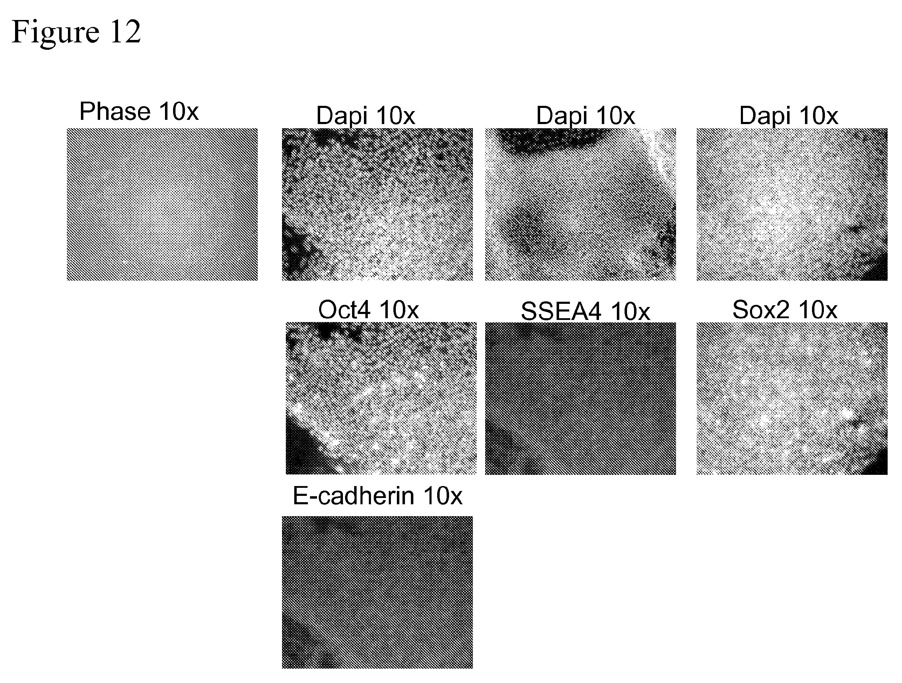

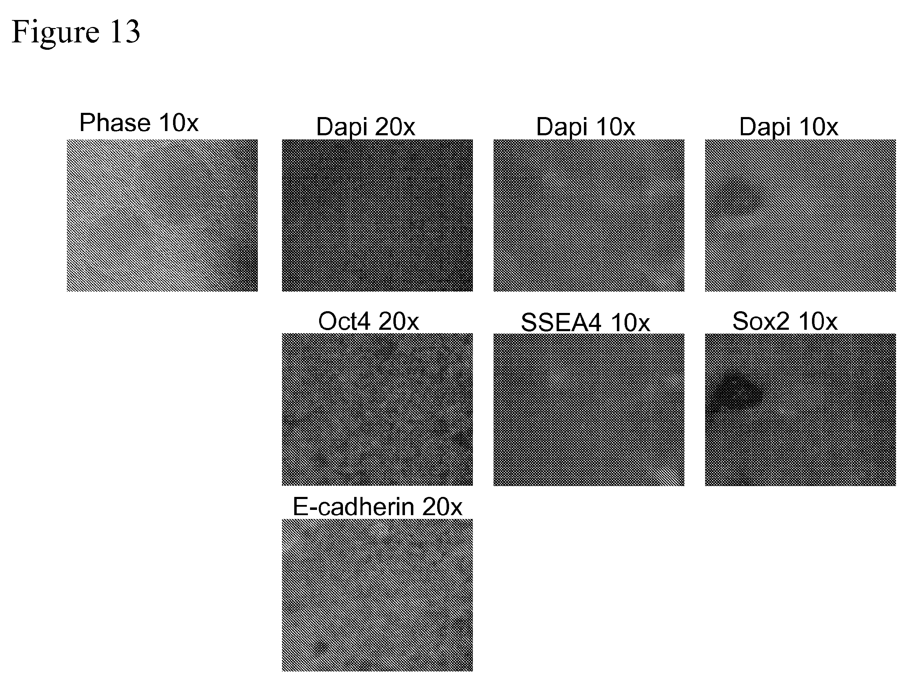

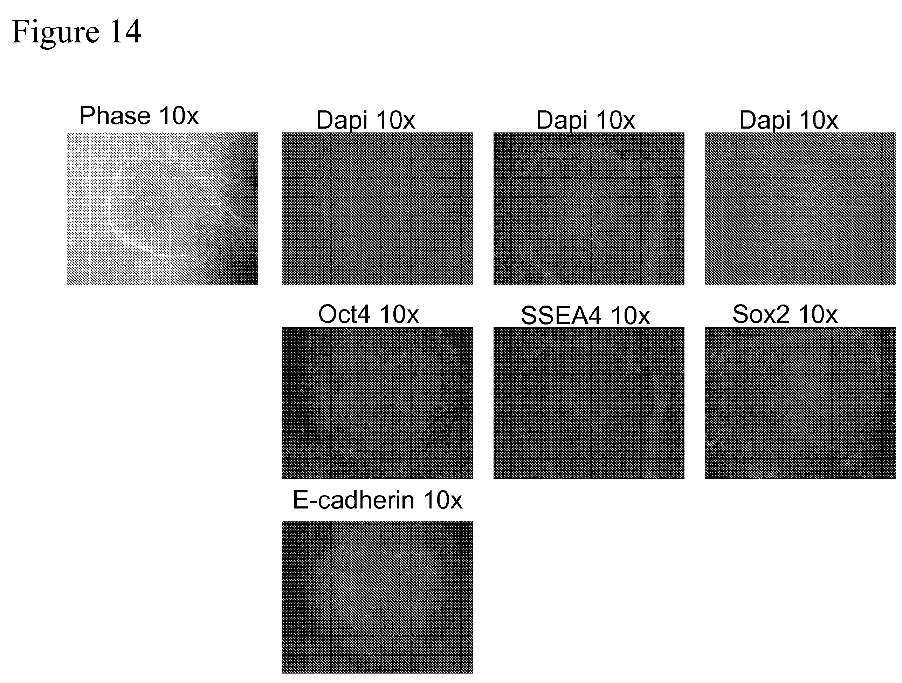

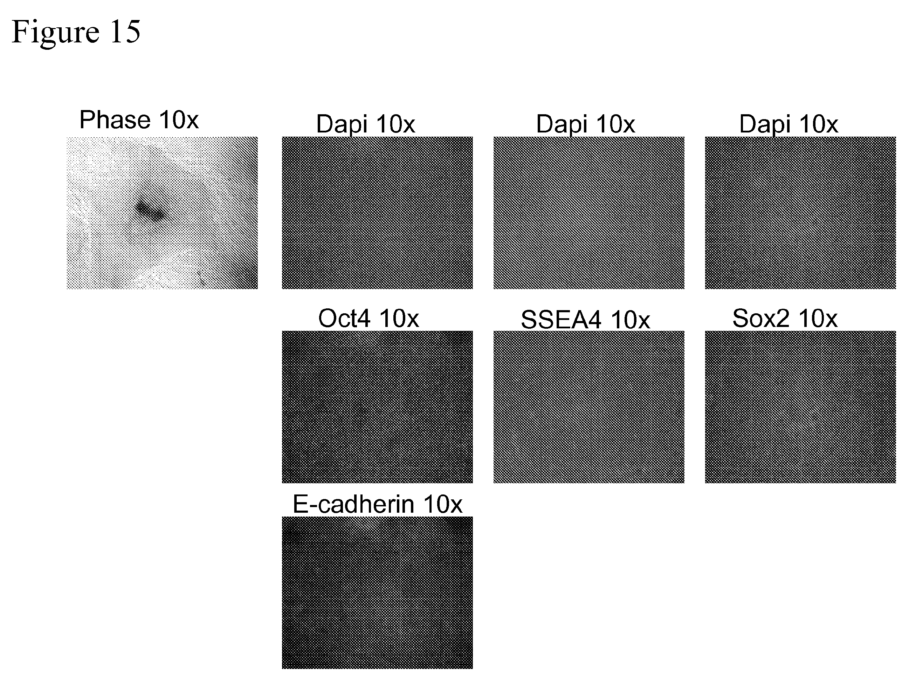

Embryonic stem cell cultures maintained on amniotic fluid derived cell feeder layers for 5-17 passages were karyotypically stable and exhibited the characteristic morphology of embryonic stem cell colonies and maintained expression of the pluripotency markers E-cadherin, Oct4, SSEA4, and Sox2 as measured by immunofluoresence **(****Figures 12-15****).** Furthermore, as measured by flow cytometric analysis, embryonic stem cell cultures maintained on amniotic fluid derived cell feeder layers for 5-17 passages were positive for the pluripotency markers SSEA-3, SSEA-4, TRA-1-60, and TRA-1-81 **(Table XII).** Embryonic stem cell cultures were also harvested for RNA and genes expression was determined by real-time PCR. Expression of genes characteristic of pluripotency such as telomerase, alkaline phosphatase, E-Cadherin, UTF-1, and Sox2 was maintained while genes characteristic of differentiation such as MyoD and Sox7 were not elevated. These results indicate that human embryonic stem cells cultured on an amniotic fluid derived cell feeder layer maintain human embryonic stem cells pluripotency. **(Table XIII).**

### Reference Example 7

### Culture of Human Embryonic Stem Cells Using Postpartum Cells Isolated from Umbilical Cord Tissue as a Feeder cell layer Maintains Markers of Human Embryonic Stem Cells.

Postpartum cells isolated from umbilical cord tissue were inactivated by mitomycin-C treatment as described above and seeded into gelatin-coated tissue culture plates at 10,000 - 30,000/cm² in DMEM-F12 or AMNIOMAX supplemented with 10% FBS and 1% non-essential amino acids and allowed to attach overnight or frozen in 10% DMSO/90% FBS and thawed at a later time. Plates of inactivated umbilical tissue derived cells were washed with PBS and seeded with embryonic stem cell in hESC medium. The embryonic stem cell cultures were passaged onto freshly seeded inactivated umbilical tissue derived cells in hESC medium for up to 14 passages. Human embryonic stem cells were examined for morphology, and expression of Sox2, Oct4, SSEA-1, SSEA-3, SSEA-4, TRA-1-60, and TRA-1-81. Karyotypic analysis was also performed.

Embryonic stem cell cultures maintained on cell feeder layers from postpartum cells isolated from umbilical cord tissue were karyotypically stable after 12 passages and exhibited the characteristic morphology of embryonic stem cell colonies and maintained expression of the pluripotency markers E-cadherin, Oct4, SSEA4, and Sox2 as measured by immunofluoresence **(****Figure 16****).** Furthermore, as measured by flow cytometric analysis, embryonic stem cell cultures maintained on cell feeder layers from postpartum cells isolated from umbilical cord tissue for 14 passages were positive for the pluripotency markers SSEA-3, SSEA-4, TRA-1-60, and TRA-1-81 **(Table XII).** Embryonic stem cell cultures were also harvested for RNA and genes expression was determined by real-time PCR. Expression of genes characteristic of pluripotency such as telomerase, alkaline phosphatase, E-Cadherin, UTF-1, and Sox2 was maintained while genes characteristic of differentiation such as MyoD and Sox7 were not elevated. These results indicate that human embryonic stem cells cultured on an amniotic fluid derived cell feeder layers maintain human embryonic stem cells pluripotency. **(Table XIII).**

### Reference Example 8

### Pluripotency of Human Embryonic Stem Cells Cultured in Amniotic Fluid-Derived Cell Conditioned Medium

*Evaluation of pluripotency via Embryoid body formation:* Human embryonic stem cells were cultured on AF AF-CM for 3-10 passages and then used to make embryoid bodies. Embryoid bodies created by scraping or enzymatic detachment of cell clusters from the culture plate will be seeded into low-attachment plastic dishes in DMEM-HG supplemented with 10% FBS and 1% non-essential amino acids and cultured for up to 14 days with media change every 3 days. Embryoid bodies formed by this method will be harvested for RNA and evaluated by real-time PCR for expression of genes of endoderm, ectoderm, and mesoderm. Alternately, the embryoid bodies will be cultured in suspension for 4 days and then plated onto tissue culture plastic and allowed to adhere and differentiate in DMEM-HG supplemented with 10% FBS and 1% non-essential amino acids for 14 days. Differentiated cells will be harvested for RNA and evaluated by real-time PCR for expression of genes of endoderm, ectoderm, and mesoderm.

### Reference Example 9

### Pluripotency of Human Embryonic Stem Cells Cultured Using Conditioned Medium from Postpartum Cells Isolated from Umbilical Cord Tissue

*Evaluation of pluripotency via embryoid body formation:* Human embryonic stem cells will be cultured on UTC-CM for 3-10 passages and used to make embryoid bodies. Embryoid bodies created by scraping or enzymatic detachment of cell clusters from the culture plate will be seeded into low-attachment plastic dishes in DMEM-HG supplemented with 10% FBS and 1% non-essential amino acids and cultured for up to 14 days with media change every 3 days. Embryoid bodies formed by this method will be harvested for RNA and evaluated by real-time PCR for expression of genes of endoderm, ectoderm, and mesoderm. Alternately, the embryoid bodies will be cultured in suspension for 4 days and then plated onto tissue culture plastic and allowed to adhere and differentiate in DMEM-HG supplemented with 10% FBS and 1% non-essential amino acids for 14 days. Differentiated cells will be harvested for RNA and evaluated by real-time PCR for expression of genes of endoderm, ectoderm, and mesoderm.

### Reference Example 10

### Pluripotency of Human Embryonic Stem Cells Cultured on an Amniotic Fluid-Derived Feeder Cell Layer

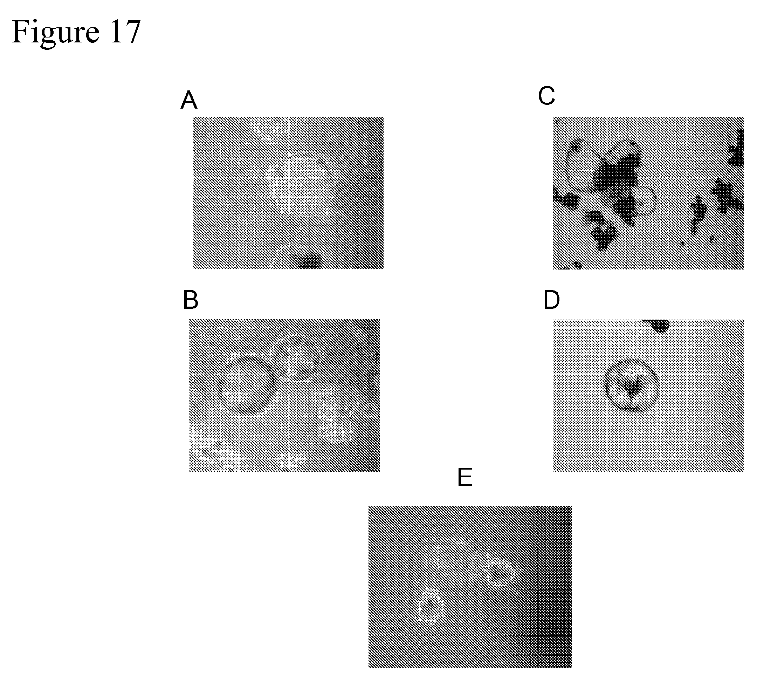

*Evaluation of pluripotency via embryoid body formation:* Human embryonic stem cells were cultured on an AF feeder cell layer for 7-14 passages prior to embryoid body formation. Embryoid bodies were created by scraping or enzymatic detachment of cell clusters from the culture plate and seeded into low-attachment plastic dishes in DMEM-F12 supplemented with 20% FBS. **Figure 17** shows embryoid bodies formed according to the methods of the present invention. Embryoid bodies were cultured for 14 days, with media changes every 3 days. Embryoid bodies were harvested for RNA and the expression of endoderm, ectoderm, and mesoderm genes was determined by real-time PCR. Expression of AFP, brachyury, HNF-3Beta, myoD1, nestin, NeuroD and Sox-17 was observed. These data indicate that human embryonic stem cells cultured on AF feeder cell layers are pluripotent and are capable of forming all three germ layers **(Table XIV).**

### Reference Example 11

### Pluripotency of Human Embryonic Stem Cells Cultured Using Postpartum Cells Isolated from Umbilical Cord Tissue as a Feeder cell layer

*Evaluation of pluripotency via embryoid body formation:* Human embryonic stem cells were cultured using postpartum cells isolated from umbilical cord tissue as a feeder cell layer for 12 passages prior to embryoid body formation. Embryoid bodies were created by scraping or enzymatic detachment of cell clusters from the culture plate and seeded into low-attachment plastic dishes in DMEM-F12 supplemented with 20% FBS. **Figure 17** shows embryoid bodies formed according to the methods of the present invention. Embryoid bodies were cultured for 14 days, with media changes every 3 days. These data indicate that human embryonic stem cells cultured using postpartum cells isolated from umbilical cord tissue as a feeder cell layer are pluripotent and are capable of forming embryoid bodies which are composed of all three germ layers.

### Reference Example 12

### Differentiation of Human Embryonic Stem cells Cultured in Amniotic Fluid-Derived Cell Conditioned Medium

Human embryonic stem cells cultured in AF cell conditioned medium for 3 to 10 passages will be subjected to differentiation protocols for cells of endodermal, ectodermal, β-cell lineage, intestinal cell lineage, or mesodermal lineage. Differentiation will be evaluated by real-time PCR for expression of genes of those lineages.

### Reference Example 13

### Differentiation of Human Embryonic Stem cells Cultured in Media Conditioned by Postpartum Cells Isolated from Umbilical Cord Tissue

Human embryonic stem cells were cultured on UTC cell feeder layers for 3 passages and treated to a differentiation protocol to produce definitive endoderm. In order to produce definitive endoderm human embryonic stem cells were grown in media conditioned by postpartum cells isolated from umbilical cord tissue and then treated DMEM-F12 medium supplemented with 0.5% FBS, 100ng/ml Activin A, and 20ng/ml Wnt3A for two days and then 2.0% FBS with 100ng/ml Activin A for 2 more day. Flow cytometric analysis of these cells for the definitive endoderm marker, CXCR4, indicated that this treatment resulted in 25% of the cells becoming positive for endoderm. These cells were also harvested for RNA and gene expression was determined by real-time PCR. Expression of the definitive endoderm markers (Sox17, Goosecoid, CXCR4, and HNF-3beta) was upregulated in the differentiated cells **(Table XV).** These data indicate that culturing embryonic stem cells in media conditioned by postpartum cells isolated from umbilical cord tissue maintains their ability to differentiate to definitive endoderm.

### Reference Example 14

### Differentiation of Human Embryonic Stem Cells Cultured on Amniotic Fluid-Derived Cell Feeder Layers

Human embryonic stem cells were cultured on AF cell feeder layers for 5 to 17 passages and treated to a differentiation protocol to produce definitive endoderm. In order to produce definitive endoderm human embryonic stem cells were grown on amniotic fluid derived cell feeder layers and treated with RPMI 1640 medium supplemented with 100nM BIO compound, 100ng/ml Activin A, and 20ng/ml Wnt3A for two days and then 0.2% FBS with 100ng/ml Activin A for an additional day. Cells were measured by flow cytometric analysis for the definitive endoderm marker, CXCR4, and were found to be positive **(Table XVI).** These cells were also harvested for RNA and gene expression was determined by real-time PCR. Expression of the definitive endoderm markers (Sox17, Goosecoid, CXCR4, and HNF-3beta) was upregulated in the differentiated cells **(Table XVII).** These data indicate that culturing embryonic stem cells on amniotic fluid derived cell feeder layers maintains their ability to differentiate to definitive endoderm.

### Reference Example 15

### Differentiation of Human Embryonic Stem Cells Cultured Using Postpartum Cells Isolated from Umbilical Cord Tissue as a Feeder cell layer

Human embryonic stem cells were cultured on a feeder cell layer of postpartum cells isolated from umbilical cord tissue for 14 passages and treated to a differentiation protocol to produce definitive endoderm. In order to produce definitive endoderm human embryonic stem cells were grown on a feeder cell layer of postpartum cells isolated from umbilical cord tissue for 14 passages and treated with RPMI 1640 medium supplemented with 100nM BIO compound, 100ng/ml Activin A, and 20ng/ml Wnt3A for two days and then 0.2% FBS with 100ng/ml Activin A for an additional day. Cells were measured by flow cytometric analysis for the definitive endoderm marker, CXCR4 and were found to be positive **(Table XVI).** These cells were also harvested for RNA and genes expression was determined by real-time PCR. Expression of the definitive endoderm markers (Sox17, Goosecoid, CXCR4, and HNF-3beta) was upregulated in the differentiated cells **(Table XVII).** These data indicate that culturing embryonic stem cells on a feeder cell layer of postpartum cells isolated from umbilical cord tissue maintains their ability to differentiate to definitive endoderm.

### Example 16

### Culture of BG01v Cells in Media Conditioned with Mouse Embryonic Fibroblasts

*Preparation of conditioned medium from Cell 1 (AF1-NF-CM) and Cell 2 (AF2-NF-CM):* Amniotic fluid-derived cells maintained in DMEM-HG supplemented with 10% FBS and 1% non-essential amino acids were incubated for 2.5hr at 37°C in 0.1ml/cm² mitomycin C (0.01mg/ml in DMEM-HG supplemented with 10% fetal bovine serum and 1% non-essential amino acids), then washed five times with PBS before detaching as single cells with TrypLE Express. Pooled cells were washed four times in DMEM-HG supplemented with 10% FBS and 1% non-essential amino acids, then seeded on gelatin-coated flasks (0.1% gelatin in water for embryo transfer, 10ml/T75, one hour to overnight at 37°C) at 56,000/cm². After attaching overnight the medium was exchanged with human embryonic stem cell medium, which did not contain bFGF, using 0.5ml/cm². Every 24h the amniotic fluid-derived cell-CM was harvested, supplemented with 2mM Glutamax, filtered, and stored at -20°C. Amniotic fluid-derived cell-CM was harvested from inactivated cultures for 7 days. The amniotic fluid-derived cell-CM was supplemented with fresh bFGF at 4ng/ml before use. The conditioned medium made in the absence of bFGF was designated AF1-NF-CM and AF2-NF-CM.

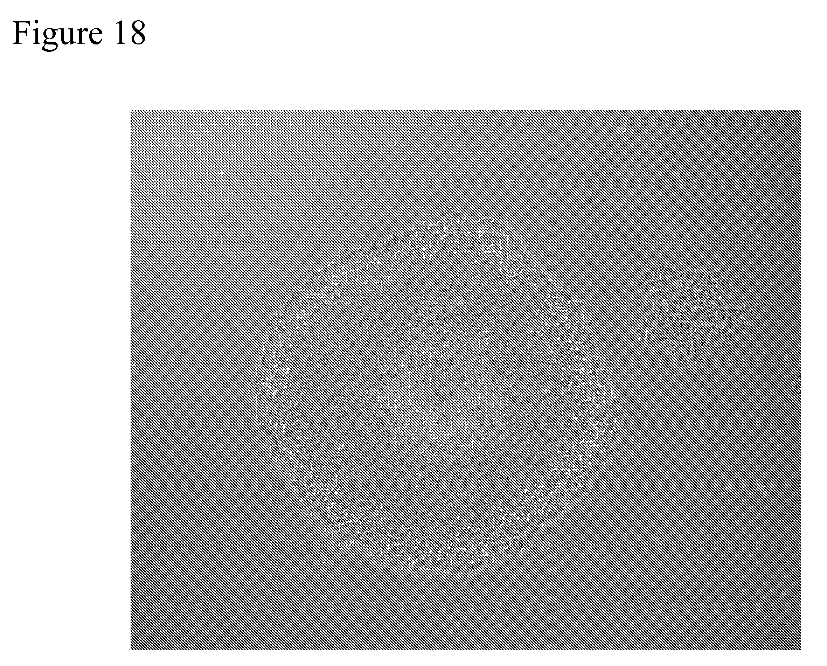

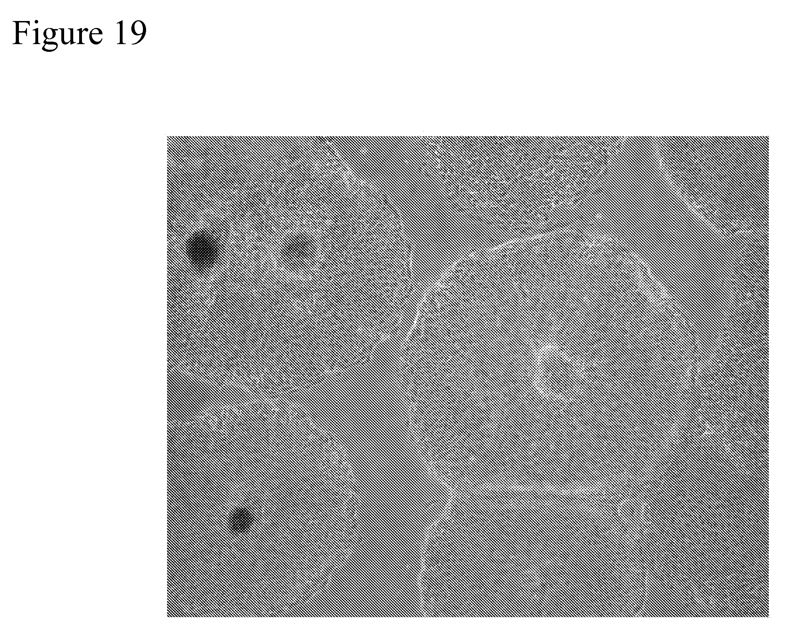

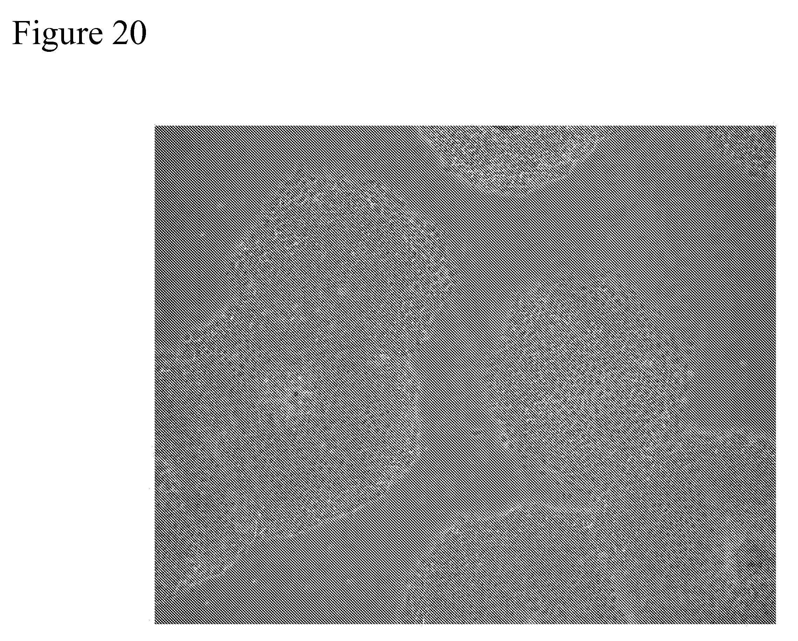

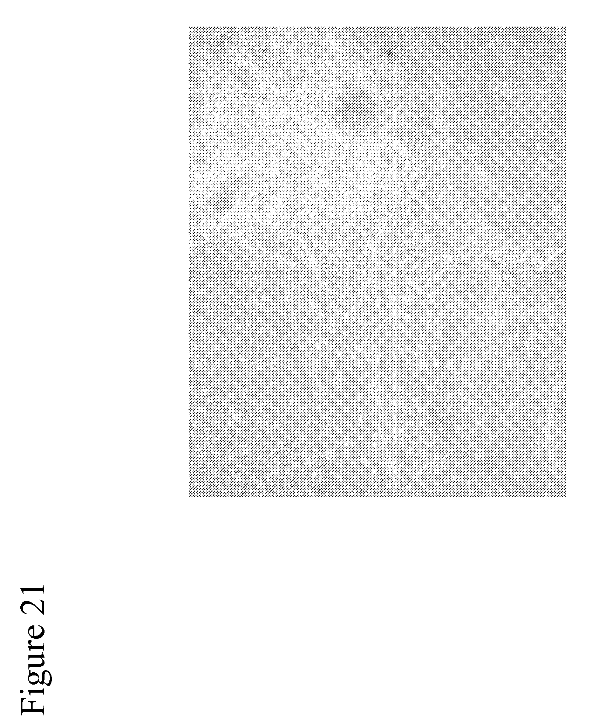

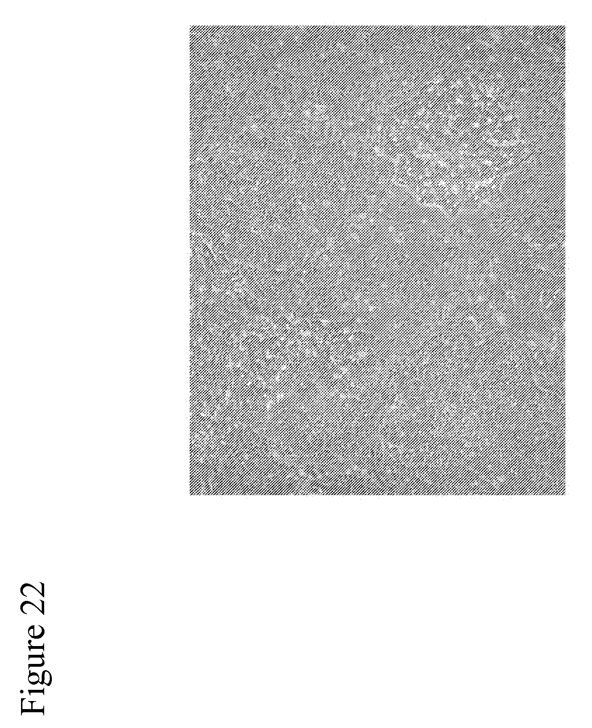

*Culture of human embryonic stem cells in amniotic fluid derived cell-conditioned medium:* On the day following passage of BG01v cells in MEF-CM on MATRIGEL when colonies had attached and begun to proliferate, amniotic fluid-derived cell-CM was substituted for MEF-CM and changed daily. Cell passage was as in the previous example, with daily feedings of amniotic fluid-derived cell -CM instead of MEF-CM. **Figure 18** shows the morphology of BG01v in MEF-CM. **Figure 19** shows the morphology of BG01v after 2 passages in AF1-NF-CM. **Figure 20** shows the morphology of BG01v after 2 passages in AF2-NF-CM. **Figure 21** shows the morphology of BG01v after 9 passages in AF1-NF-CM. **Figure 22** shows the morphology of BG01v after 10 passages in AF2-NF-CM. Differentiated cells represented a large part of the cells in the culture after 9 passages. Cell proliferation paralleled that of cells in MEF-CM and cells in AF1-CM and AF2-CM were generally passed on the same day at the same split ratio as cells in MEF-CM.

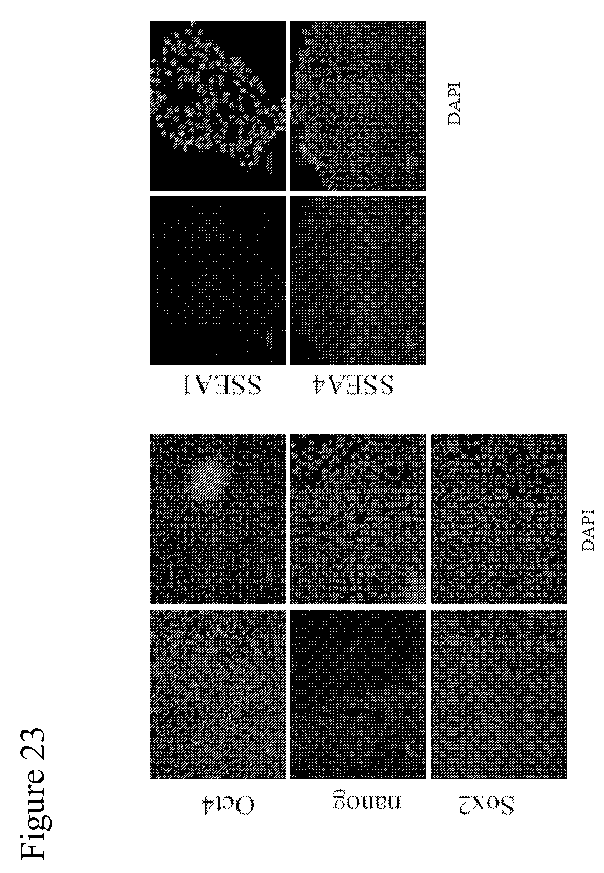

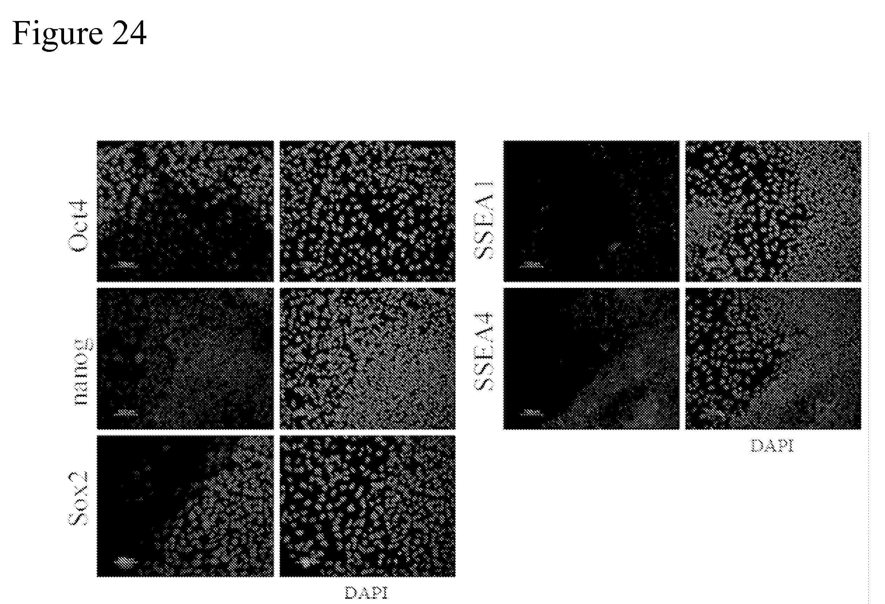

*Characterization of human embryonic stem cells on MATRIGEL in conditioned medium obtained from Cell 1 and Cell 2 in the absence of bFGF:* Cells in their 5th passage in MEF-CM and AF2-NF-CM and their 35th passage overall were evaluated by immunostaining. Cells in their fifth passage in MEF-CM, AF1-NF-CM, and AF2-NF-CM were evaluated by FACS. By FACS they were positive for SSEA-1, SSEA-3, SSEA-4, TRA-1-60, and TRA-1-81 **(Table XVIII**). By immunostaining undifferentiated cells in MEF-CM were positive for nanog, Sox2, and Oct4 **(****Figure 23****).** Undifferentiated cells in AF2-NF-CM were positive for Oct4, Sox2, and nanog, while differentiated cells in AF2-NF-CM were negative for nanog, Sox2, and Oct4 **(****Figure 24****). Table XIX** shows expression of pluripotency markers in human embryonic stem cells cultured in AF1-NF-CM and AF2-NF-CM by real-time PCR. **Table XX** shows expression of differentiation markers in human embryonic stem cells cultured in AF1-NF-CM and AF2-NF-CM by real-time PCR.

**TABLE I: CELL SURFACE MARKERS ON AMNIOTIC FLUID-DERIVED CELLS SUITABLE FOR USE IN THE PRESENT INVENTION.**

| Marker | Cell 1 | Cell 2 | Cell 3 | Cell 4 | Cell 5 | Cell 6 | Cell 7 | Cell 8 |
|---|---|---|---|---|---|---|---|---|
| CD9 | | Weak | | | | | | |
| CD10 | ++ | ++ | Weak | Weak | | | | - |
| CD105 | ++ | ++ | Weak | Weak | + | + | + | Weak |
| CD11 | | | | | | | - | - |
| CD117 | - | - | - | - | Weak | - | - | - |
| CD13 | | Weak | - | - | | + | | |
| CD24 | - | - | + | + | | | | |
| CD29 (Beta 1 integrin) | | | + | + | | | | + |
| CD31 | | | | | | | - | |
| CD34 | | | | | Weak | - | - | - |
| CD44 | + | ++ | + | + | | + | + | + |
| CD45 | | | | | | - | - | |
| CD49b (Alpha 2 integrin) | ++ | ++ | | | | + | | |
| CD49c | ++ | + | + | + | | | | |
| CD49d | - | Weak | | | | | | |
| CD49e (Alpha 5 integrin) | +++ | + | | | | | + | |
| CD49f | ++ | ++ | | | | | | |
| CD51/61 | - | - | + | + | | | | |
| CD54 | Weak | Weak | | | | | | |
| CD58 | ++ | Weak | | | | | | |
| CD61 | | - | + | + | | | | |
| CD73 | ++ | ++ | + | + | | | + | + |
| CD81 | ++ | ++ | | | | | | |
| CD166 | | | | | | | + | |
| CD90 | +++ | ++ | + | + | + | | + | + |
| CD95 | + | + | | | | | | |
| CD146 | - | + | | | | | | |
| CD184 | - | - | | | | | | |
| c-Met | | | + | + | | | | |
| E-Cadherin | - | - | | | | | | |
| EPCAM | - | - | + | + | | | | |
| EGF Receptor | | - | | | | | | |
| HLA ABC | | | + | + | + | + | + | + |
| HLA II-DR | | | | - | | | | |
| HLA D | | | | | | - | - | - |
| Oct-4 | | | | | + | + | | + |
| SSEA-1 | | | | | - | - | | |
| SSEA-3 | - | - | + | + | + | + | | |
| SSEA-4 | + | Weak | + | + | + | + | | |
| Stro-1 | | | | | | - | | |
| Telomerase | | | | | + | | | |
| TRA 1-60 | | | + | + | | + | | |
| TRA 1-81 | | | + | + | | + | | |
| TRA 1-85 | | | | | | | | |
| TRA 2-49 | | | - | - | | | | |
| TRA 2-54 | | | | | | + | | |

**TABLE II: PCR ANALYSIS OF AMNIOTIC FLUID-DERIVED CELLS SUITABLE FOR USE IN THE PRESENT INVENTION.**

| Marker | Cell 1 | Cell 2 | Cell 3 | Cell 4 |
|---|---|---|---|---|
| Oct-3 | | | - | - |
| Oct-4 | | | - | - |
| Sox-2 | | | - | - |
| Rex-1 | | | Weak | Weak |
| HTERT | | | - | - |
| Sox-17 | - | - | + | + |
| GATA-4 | | | Weak | Weak |
| GATA-6 | | | + | + |
| HNF-1beta | | | + | + |
| HNF-3beta | - | - | Weak | Weak |
| Pdx-1 | | | - | - |
| NGN-3 | | | - | - |
| Musashi-1 | | | + | + |
| Hes-1 | | | + | + |
| NeuroD-1 | | | - | - |
| Pax-4 | | | - | - |
| Pax-6 | | | + | + |
| Secretin | | | - | - |
| GIP | | | + | + |
| Glucagon | | | - | - |
| Somatostatin | | | + | + |
| CCK | | | - | - |
| Gastrin | | | - | - |
| Insulin | | | Weak | Weak |
| Nestin | | | + | + |

**TABLE III: FACS ANALYSIS OF H9 CELLS CULTURED IN MEF-CM AND IN AF CELL CONDITIONED MEDIA FOR THE INDICATED NUMBER OF PASSAGES.**

| | MEF-CM | AF1-CM p5 | AF2-CM p5 | AF3-CM p6 | AF4-CM p7 |
|---|---|---|---|---|---|
| SSEA-1 | - | - | - | - | -/+ |
| SSEA-3 | + | + | + | + | + |
| SSEA-4 | + | + | + | + | + |
| TRA-1-60 | + | + | + | + | + |
| TRA-1-81 | + | + | + | + | + |

**TABLE IV: DISTRIBUTION OF CHROMOSOME NUMBERS IN 100 METAPHASES ANALYZED FOR H9 P82 IN MEF-CM.**

| Number of chromosomes | Number of metaphases |
|---|---|
| 44 | 2 |
| 45 | 3 |
| 46 | 92 |
| 47 | 3 |

**TABLE V: EXPRESSION OF PLURIPOTENCY MARKERS BY H9 CULTURED IN AF1-CM AND AF2-CM.**

| | AF1-CM p3 | AF1-CM p4 | AF1-CM p5 | AF2-CM p3 | AF2-CM p4 | AF2-CM p5 |
|---|---|---|---|---|---|---|
| ABCG2 | 1.85 | 2.42 | 0.95 | 1.12 | 2.22 | 1.19 |
| CFC-1 | 4.23 | 0.66 | 2.86 | 4.77 | 1.01 | 2.53 |
| Fox D3 | 5.87 | 4.51 | 1.66 | 5.92 | 5.04 | 1.9 |
| GJA1 | 2.76 | 1.26 | 1.48 | 2.85 | 1.6 | 1.88 |
| GJA7 | 2.6 | 5.83 | 1.19 | 1.99 | 4.67 | 1.24 |
| Oct4 | 1.66 | 4.08 | 0.87 | 1.25 | 3.12 | 0.98 |
| Sox2 | 5.8 | 9.13 | 2.34 | 1.72 | 3.19 | 1.52 |
| Tert | 2.04 | 2.25 | 1.99 | 1.95 | 2.75 | 2.04 |
| UTF-1 | 3.51 | 2.63 | 3.31 | 5.28 | 1.52 | 2.72 |
| ZFP42 | 2.05 | 1.73 | 1.29 | 1.54 | 1.63 | 1.32 |

Numbers represent fold expression relative to H9 cells cultured in MEF-CM.

**TABLE VI: EXPRESSION OF DIFFERENTIATION MARKERS BY H9 CULTURED IN AF1-CM AND AF2-CM.**

| | AF1-CM p3: | AF1-CM p4 | AF1-CM p5 | AF2-CM p3 | AF2-CM p4 | AF2-CM p5 |
|---|---|---|---|---|---|---|
| AFP | ND | ND | ND | ND | ND | ND |
| Fox2 | 3.77 | 7.15 | 0.329 | 5.18 | 6.7 | 1.05 |
| GATA2 | 5.82 | 6.32 | 1.28 | 5.97 | 9.92 | 1.36 |
| GATA4 | 98.17 | ND | 26.44 | 47.9 | 70.68 | 59.91 |
| IPF-1 | ND | ND | ND | ND | ND | ND |
| KRT-15 | 3.04 | 4.5 | 0.27 | 0.98 | 6.67 | 0.43 |
| msx-1 | 7.53 | ND | ND | 6.21 | ND | 6.49 |
| myoD1 | ND | ND | 10.31 | ND | ND | ND |
| nestin | 3.07 | 7.69 | 1.01 | 2.48 | 6.43 | 1.16 |
| Sox1 | ND | ND | ND | ND | ND | ND |
| T-box | 19.62 | ND | 17.36 | 28.63 | 24.98 | 10.03 |
| Tubb3 | 4.79 | 11.36 | 2.27 | 2.95 | 9.13 | 2.33 |
| vimentin | 2.62 | 6.12 | 0.88 | 1.93 | 5.59 | 0.93 |

Numbers represent fold expression relative to H9 cultured in MEF-CM. ND, not detected.

**TABLE VII: DISTRIBUTION OF CHROMOSOME NUMBERS IN 100 METAPHASES ANALYZED FOR H9 AFTER 9 PASSAGES IN AF3-CM.**

| Number of chromosomes | Number of metaphases |
|---|---|
| 44 | 1 |
| 45 | 9 |
| 46 | 88 |
| 47 | 2 |

**TABLE VIII: DISTRIBUTION OF CHROMOSOME NUMBERS IN 100 METAPHASES ANALYZED FOR H9 AFTER 9 PASSAGES IN AF4-CM.**

| Number of chromosomes | Number of metaphases |
|---|---|
| 44 | 4 |
| 45 | 20 |
| 46 | 71 |
| 47 | 5 |

**TABLE IX: EXPRESSION OF PLURIPOTENCY MARKERS BY H9 CULTURED IN AF3-CM AND AF4-CM.**

| | AF3-CM p2 | AF3-CM p3 | AF3-CM p4 | AF4-CM p2 | AF4-CM p3 | AF4-CM p4 |
|---|---|---|---|---|---|---|
| ABCG2 | 1.26 | 2.01 | 1.31 | 1.33 | 1.75 | 1.12 |
| CFC-1 | 3.28 | 1.54 | 4.3 | 5.7 | 2.13 | 4.48 |
| Fox D3 | 2.42 | 1.63 | 0.91 | 1.99 | 1.33 | 1.26 |
| GJA1 | 1.81 | 1.82 | 1.69 | 2 | 2.39 | 1.53 |
| GJA7 | 0.85 | 1.93 | 0.94 | 1.11 | 1.55 | 1.04 |
| Oct4 | 0.86 | 1.31 | 0.76 | 0.86 | 1.15 | 0.86 |
| Sox2 | 1.26 | 1.72 | 0.45 | 1.33 | 0.88 | 0.96 |
| Tert | 1.08 | 1.47 | 0.94 | 1.07 | 1.29 | 1.17 |
| UTF-1 | 2.34 | 1.8 | 1.8 | 3.24 | 2.7 | 2.52 |
| ZFP42 | 0.99 | 1.16 | 1.07 | 1.14 | 1.04 | 1.28 |

**TABLE X: EXPRESSION OF DIFFERENTIATION MARKERS BY H9 CULTURED IN AF3-CM AND AF4-CM.**

| | AF3-CM p2 | AF3-CM p3 | AF3-CM p4 | AF4-CM p2 | AF4-CM p3 | AF4-CM p4 |
|---|---|---|---|---|---|---|
| AFP | 34.5 | ND | ND | ND | 41.52 | ND |
| FoxA2 | 10.92 | 2.33 | 8.54 | 9.3 | 7.24 | 12.25 |
| GATA2 | 1.76 | 1.79 | 3.31 | 3.13 | 1.35 | 1.67 |
| GATA4 | 1.67 | 5 | 1.89 | 3.38 | 6.08 | 2.86 |
| IPF-1 | ND | ND | ND | ND | ND | ND |
| KRT-15 | 1.77 | 8.21 | 2.93 | 4.55 | 8.14 | 2.66 |
| msx-1 | 1.24 | 3.78 | ND | 4.83 | 7.29 | 1.6 |
| myoD1 | 7.56 | ND | ND | ND | ND | ND |
| nestin | 1.03 | 2.27 | 1.02 | 1.4 | 2.11 | 1.39 |
| Sox1 | ND | ND | ND | 2.42 | ND | ND |
| T-box | 1.08 | 1.52 | 1.33 | 1.31 | ND | 0.23 |
| Tubb3 | 1.33 | 3.46 | 1.44 | 2.24 | 3.22 | 2.07 |
| vimentin | 1.06 | 3.65 | 1.05 | 1.49 | 3.52 | 1.18 |

**TABLE XI: EMBRYONIC STEM CELL EXPRESSION OF PLURIPOTENCY AND DIFFERENTIATION MARKERS AS MEASURED BY REAL TIME PCR.**

| | **Brachyury** | **N-Cadherin** | **CXCR4** | **E-Cadherin** | **Goosecoid** | **HNF-3b** | **OCt4** | **SOX-17** | **Gata-4** | **SOX-7** |
|---|---|---|---|---|---|---|---|---|---|---|
| H9 p48 grown in MEF-CM | 1.0 | 1.0 | 1.0 | 1.0 | 1.0 | 1.0 | 1.0 | 1.0 | 1.0 | 1.0 |
| H9 p48 grown 3 passages in UTC-CM | 0.1 | 1.5 | 0.8 | 1.0 | 2.9 | 1.1 | 1.1 | 1.1 | 1.1 | 1.6 |

**TABLE XII: EMBRYONIC STEM CELL EXPRESSION OF PLURIPOTENCY MARKERS AS MEASURED BY FACS.**

| | **UTC p12p50** | **AF3 p5p46** | **AF4 p5p46** | **AF1 p14p50** | **AF2 p14p50** | **H9p37** | **H9p48** |
|---|---|---|---|---|---|---|---|
| Tra 1-60 | 99.2 | 98.6 | 91.6 | 95.6 | 94.1 | 100.0 | 99.9 |
| Tra 1-81 | 99.6 | 99.2 | 93.6 | 96.0 | 93.0 | 100.0 | 100.0 |
| SSEA1 | 77.2 | 45.6 | 67.8 | 44.7 | 42.0 | 89.7 | 63.6 |
| SSEA3 | N/A | 43.0 | 41.5 | 98.2 | 85.9 | 59.4 | 99.9 |
| SSEA4 | 88.3 | 63.4 | 60.4 | 81.1 | 88.9 | 78.2 | 99.5 |
| CD56 | 16.3 | 28.6 | 29.1 | 7.5 | 23.9 | 29.9 | 9.3 |
| E-Cadherin | 63.1 | 73.2 | 55.1 | 69.6 | 81.7 | 81.2 | 91.4 |
| CD9 | 84.3 | 66.1 | 55.8 | 79.6 | 90.3 | 81.5 | 96.8 |
| CD184 | 2.4 | 3.1 | 3.5 | 0.7 | 1.7 | 0.7 | 0.3 |
| CD117 | 71.8 | 66.5 | 69.7 | 77.4 | 69.2 | 97.1 | 97.9 |
| PDGF-R | N/A | 18.6 | 9.0 | 6.8 | 10.9 | 14.4 | 42.1 |

**TABLE XIII: EMBRYONIC STEM CELLS EXPRESSION OF PLURIPOTENCY MARKERS AS MEASURED BY REAL TIME PCR.**

| | UTC p12p50 | AF3 p5p46 | AF4 p5p46 | AF1 p14p50 | AF2 p14p50 | H9p37 | H9p48 |
|---|---|---|---|---|---|---|---|
| Oct4 | 1.206 | 2.514 | 4.525 | 3.185 | 4.899 | 1 | 4.611 |
| Telomerase | 0.887 | 1.107 | 1.559 | 0.954 | 1.051 | 1 | 1.786 |
| Alkaline Phosphatase | 1.494 | 1.685 | 2.265 | 0.848 | 1.268 | 1 | 1.496 |
| E-Cadherin | 1.911 | 0.912 | 1.26 | 0.92 | 0.985 | 1 | 1.199 |
| UTF-1 | 0.788 | 0.498 | 0.404 | 0 | 0.653 | 1 | 0.506 |
| MyoD | 0.579 | 2.518 | 3.4 | 0.821 | 0.671 | 1 | 0.173 |
| Sox2 | 0.358 | 0.817 | 0.676 | 0.349 | 0.791 | 1 | 0.496 |
| Sox7 | 1.017 | 2.149 | 1.908 | 0.862 | 1.923 | 1 | 0.211 |

**TABLE XIV: FOLD DIFFERENCE IN GENE EXPRESSION OF EMBRYOID BODIES DERIVED FROM H9 HES CELLS GROWN ON INDICATED FEEDER VERSUS PASSAGE 50 H9 HES CELLS GROWN ON MATRIGEL IN MEF CONDITIONED MEDIA.**

| | AFP | Brachyury | GAPDH | HNF-3b | MyoD1 | Nestin | Neuro D | Sox17 |
|---|---|---|---|---|---|---|---|---|
| AF1 | 91164 | 130 | 1 | 1143 | 34 | 1 | 697 | 22 |
| AF2 | 46863 | 3 | 1 | 383 | 32 | 2 | 340 | 12 |
| AF3 | 2081471 | 7 | 1 | 5530 | 68 | 6 | 3812 | 90 |
| AF4 | 1573092 | 39 | 1 | 2662 | 81 | 6 | 1159 | 62 |

**TABLE XV: EMBRYONIC STEM CELLS CULTURED IN MEDIA CONDITIONED BY POSTPARTUM CELLS ISOLATED FROM UMBILICAL CORD TISSUE MAINTAIN THEIR ABILITY TO DIFFERENTIATE TO DEFINITIVE ENDODERM.**

| | Brachyury | CFC-1 | CXCR4 | E-Cadherin | Goosecoid | HNF3b | Oct4 | Sox17 | Sox2 | Sox7 |
|---|---|---|---|---|---|---|---|---|---|---|
| p3 in UCC-CM | 1.0 | 1.0 | 1.0 | 1.0 | 1.0 | 1.0 | 1.0 | 1.0 | 1.0 | 1.0 |
| p3 in UCC-CM Differentiated | 7.1 | 13.2 | 58.5 | 0.7 | 24.1 | 928.7 | 1.0 | 432.0 | 0.7 | 0.8 |

**TABLE XVI: DIFFERENTIATION OF EMBRYONIC STEM CELLS TO DEFINITIVE ENDODERM AS MEASURED BY PERCENT OF CD184 (CXCR4) POSITIVE CELLS DETECTED BY FACS.**

| Feeder Cell | AF3 | AF4 | AF1 | AF2 | Umbilical Cord Tissue Cells |
|---|---|---|---|---|---|
| % CXCR4 Positive | 35 | 15 | 74 | 51 | 10 |

**TABLE XVII: DIFFERENTIATION OF EMBRYONIC STEM CELLS MAINTAINED ON AN AMNIOTIC FLUID DERIVED FEEDER CELL LAYER OR ON A FEEDER LAYER OF POSTPARTUM CELLS ISOLATED FROM UMBILICAL CORD TISSUE TO DEFINITIVE ENDODERM AS MEASURED BY EXPRESSION OF TRANSCRIPTION FACTORS DETECTED BY REAL TIME PCR.**

| | Feeder Free | AF3 | AF4 | AF1 | AF2 | Umbilical Cord Tissue Cells |
|---|---|---|---|---|---|---|
| Brachyury | 1 | 1 | 2 | 1 | 1 | 6 |
| CXCR4 | 1 | 35 | 19 | 56 | 37 | 13 |
| Goosecoid | 1 | 33 | 30 | 51 | 107 | 69 |
| HNF3b | 1 | 110 | 57 | 103 | 154 | 52 |
| Oct4 | 1 | 0.08 | 0.04 | 0.01 | 0.06 | 0.04 |
| Sox17 | 1 | 194 | 128 | 269 | 481 | 296 |

**TABLE XVIII: FACS ANALYSIS OF BG01V CULTURED IN MEF-CM, AF1-NF-CM, AND AF2-NF-CM FOR 5 PASSAGES.**

| | MEF-CM | AF1-NF-CM | AF2-NF-CM |
|---|---|---|---|
| SSEA-1 | + | + | + |
| SSEA-3 | + | + | +/- |
| SSEA-4 | + | + | + |
| TRA-1-60 | + | + | + |
| TRA-1-81 | + | + | + |

**TABLE XIX: EXPRESSION OF PLURIPOTENCY MARKERS BY BG01V CULTURED IN AF1-NF-CM AND AF2-NF-CM.**

| | p9 in AF1-NF-CM | p10 in AF2-NF-CM |
|---|---|---|
| ABCG2 | 1.89 | 1.45 |
| CFC1 | 1.7 | 0.9 |
| FoxD3 | 0.18 | 0.09 |
| GJA1 | 0.75 | 0.91 |
| GJA7 | 0.77 | 0.74 |
| hTERT | 1.18 | 0.92 |
| Oct3/4 | 0.27 | 0.18 |
| Sox2 | 1.25 | 1.25 |
| UTF1 | 3.73 | 1.43 |
| ZFP42 | 0.47 | 0.4 |

**TABLE XX: EXPRESSION OF DIFFERENTIATION MARKERS BY BG01V CULTURED IN AF1-NF-CM AND AF2-NF-CM.**

| p9 | p9 in AF1-NF-CM | p10 in AF2-NF-CM |
|---|---|---|
| Tubb3 | 1.45 | 1.37 |
| FoxA2 | 1.76 | 4.75 |
| GATA-2 | 1.11 | 0.52 |
| GATA-4 | 1.53 | 0.97 |
| IPF-1 | 0.69 | 0.7 |
| KRT-15 | 1.17 | 2.79 |
| msx-1 | 25.42 | 35.61 |
| myoD1 | 3.87 | 1.3 |
| nestin | 0.82 | 1.09 |
| Sox-1 | 0.58 | 2.94 |
| T | 1.81 | 1.71 |
| vim | 2.11 | 4.22 |

## Claims

1. A method to generate conditioned medium for the propagation of pluripotent stem cells, comprising the steps of:
(a) culturing cells that supply the conditioning factors,
(b) adding a base medium to the cells that supply the conditioning factors,
(c) exposing the base medium to the cells that supply the conditioning factors for a period of time sufficient to condition the medium, and
(d) removing the conditioned medium;
wherein the cells that supply the conditioning factors are amniotic fluid-derived cells and express at least one of the following markers: HNF-1beta, HNF-3beta, SOX- 17, or GATA 6.

2. The method of claim 1, wherein the pluripotent stem cells are undifferentiated pluripotent stem cells.

3. The method of claim 1 or claim 2, wherein the cells that supply the conditioning factors are inactivated.

4. The method of claim 1 or claim 2, wherein the conditioned medium is purified after removal from the cells that supply the conditioning factors.

5. The method of any one of the preceding claims, wherein the amniotic fluid-derived cells do not express at least one of the following markers: CD 117, Oct-4, or Tra2-54.

6. The method of any one of the preceding claims, wherein the amniotic fluid-derived cells do not express at least one of the following markers: HNF-3beta, or SOX- 17.

## Patentansprüche

1. Verfahren zur Erzeugung von konditioniertem Medium für die Vermehrung von pluripotenten Stammzellen, umfassend die Schritte:
(a) Kultivieren von Zellen, die die Konditionierungsfaktoren bereitstellen,
(b) Hinzufügen eines Basismediums zu den Zellen, die die Konditionierungsfaktoren bereitstellen,
(c) Exprimieren des Basismediums gegenüber den Zellen, die die Konditionierungsfaktoren bereitstellen, über einen Zeitraum, der ausreicht, um das Medium zu konditionieren, sowie
(d) Entfernen des konditionierten Mediums;
wobei es sich bei den Zellen, die die Konditionierungsfaktoren bereitstellen, um Zellen, die von Amnionflüssigkeit abgeleitet sind und die mindestens einen der folgenden Marker exprimieren: HNF-1beta, HNF-3beta, SOX- 17 oder GATA 6, handelt.

2. Verfahren nach Anspruch 1, wobei es sich bei den pluripotenten Stammzellen um undifferenzierte pluripotente Stammzellen handelt.

3. Verfahren nach Anspruch 1 oder Anspruch 2, wobei die Zellen, die die Konditionierungsfaktoren bereitstellen, inaktiviert werden.

4. Verfahren nach Anspruch 1 oder Anspruch 2, wobei das konditionierte Medium nach dem Entfernen von den Zellen, die die Konditionierungsfaktoren bereitstellen, aufgereinigt wird.

5. Verfahren nach einem der vorhergehenden Ansprüche, wobei die von Amnionflüssigkeit abgeleiteten Zellen nicht mindestens einen der folgenden Marker exprimieren: CD117, Oct-4 oder Tra2-54.

6. Verfahren nach einem der vorhergehenden Ansprüche, wobei die von Amnionflüssigkeit abgeleiteten Zellen nicht mindestens einen der folgenden Marker exprimieren: HNF-3beta oder SOX- 17.

## Revendications

1. Procédé de production d'un milieu conditionné pour la propagation de cellules souches pluripotentes, comprenant les étapes suivantes:
(a) cultiver des cellules qui fournissent les facteurs de conditionnement,
(b) ajouter un milieu de base aux cellules qui fournissent les facteurs de conditionnement,
(c) exposer le milieu de base aux cellules qui fournissent les facteurs de conditionnement pendant une période de temps suffisante pour conditionner le milieu, et
(d) retirer le milieu conditionné;
dans lequel les cellules qui fournissent les facteurs de conditionnement sont des cellules dérivées du liquide amniotique et expriment au moins un des marqueurs suivants: HNF-1bêta, HNF-3bêta, SOX- 17, ou GATA 6.

2. Procédé selon la revendication 1, dans lequel les cellules souches pluripotentes sont des cellules souches pluripotentes indifférenciées.

3. Procédé selon la revendication 1 ou la revendication 2, dans lequel les cellules qui fournissent les facteurs de conditionnement sont inactivées.

4. Procédé selon la revendication 1 ou la revendication 2, dans lequel le milieu conditionné est purifié après son retrait des cellules qui fournissent les facteurs de conditionnement.

5. Procédé selon l'une quelconque des revendications précédentes, dans lequel les cellules dérivées du liquide amniotique n'expriment pas au moins un des marqueurs suivants: CD117, Oct-4, ou Tra2-54.

6. Procédé selon l'une quelconque des revendications précédentes, dans lequel les cellules dérivées du liquide amniotique n'expriment pas au moins un des marqueurs suivants: HNF-3bêta, ou SOX- 17.
